(19) Europäisches Patentamt
European Patent Office
Office européen des brevets

(11) Veröffentlichungsnummer: **0 172 551**
**A2**

(12) # EUROPÄISCHE PATENTANMELDUNG

(21) Anmeldenummer: 85110351.5

(22) Anmeldetag: 19.08.85

(51) Int. Cl.⁴: C 07 C 131/02
C 07 C 149/30, C 07 D 521/00
A 01 N 35/10

(30) Priorität: 22.08.84 DE 3430804
24.04.85 DE 3514798

(43) Veröffentlichungstag der Anmeldung:
26.02.86 Patentblatt 86/9

(84) Benannte Vertragsstaaten:
AT BE CH DE FR GB IT LI NL

(71) Anmelder: BAYER AG
Konzernverwaltung RP Patentabteilung
D-5090 Leverkusen 1 Bayerwerk(DE)

(72) Erfinder: Wroblowsky, Heinz-Jürgen, Dr.
Gladbacher Strasse 34
D-4018 Langenfeld(DE)

(72) Erfinder: Stetter, Jörg, Dr.
Gellertweg 4
D-5600 Wuppertal 1(DE)

(72) Erfinder: Eue, Ludwig, Dr.
Paul-Klee-Strasse 36
D-5090 Leverkusen 1(DE)

(72) Erfinder: Santel, Hans-Joachim, Dr.
Gerstenkamp 19
D-5000 Koeln 80(DE)

(72) Erfinder: Schmidt, Robert R. Dr.
Im Waldwinkel 110
D-5060 Bergisch Gladbach 2(DE)

(54) **2,4-Bis-(alkoximinoalkyl)-cyclohexan-1,3-dione.**

(57) Die Erfindung betrifft neue 2,4-Bis-(alkoximinoalkyl)-cyclohexan-1,3-dione der Formel (I)

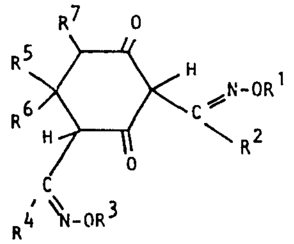

(I)

in welcher

$R^1$ und $R^3$ unabhängig voneinander jeweils für Wasserstoff, Alkyl, Alkenyl, Alkinyl, Alkoxyalkyl, Alkylthioalkyl, Halogenalkyl, Halogenalkenyl, Alkoxycarbonylalkyl, Alkoxyiminoalkyl, für jeweils gegebenenfalls substituiertes Aralkyl, Aryl oder Heteroarylalkyl oder für Tetrahydropyranyl stehen,

$R^2$ und $R^4$ unabhängig voneinander jeweils für Wasserstoff, Alkyl, Alkenyl, Alkinyl, Alkoxyalkyl, Alkylthioalkyl, Halogenalkyl, Cycloalkyl oder für jeweils gegebenenfalls substituiertes Aryl, Aryloxylalkyl oder Arylthioalkyl stehen und

$R^5$ und $R^6$ unabhängig voneinander jeweils für Wasserstoff, Alkyl, Alkenyl, Alkoxyalkyl, Alkylthioalkyl oder für jeweils gegebenenfalls substituiertes Cycloalkyl, Cycloalkenyl, Aryl, Heteroaryl, Aralkyl, Aryloxyalkyl oder Arylthioalkyl stehen und

$R^7$ für Wasserstoff steht oder gemeinsam mit $R^6$ für einen zweifach verknüpften Alkandiylrest steht,

sowie deren Metallsalze, Verfahren zur Herstellung dieser Verbindungen und ihre Verwendung als Herbizide.

EP 0 172 551 A2

BAYER AKTIENGESELLSCHAFT 5090 Leverkusen, Bayerwerk
Konzernverwaltung RP
Patentabteilung KM-klu/c
Ib

## 2,4-Bis-(alkoximinoalkyl)-cyclohexan-1,3-dione

Die Erfindung betrifft neue 2,4-Bis-(alkoximinoalkyl)-cyclohexan-1,3-dione, mehrere Verfahren zu ihrer Herstellung und ihre Verwendung als Herbizide.

Es ist bereits bekannt, daß bestimmte Cyclohexan-1,3-dione, wie beispielsweise das 2-(1-Allyloximino)-butyl-5,5-dimethyl-4-methoxycarbonyl-cyclohexan-1,3-dion-Mononatriumsalz, herbizide, insbesondere auch selektiv-herbizide Eigenschaften besitzen (vergl. z.B. DE-OS 24 39 104).

Die herbizide Wirkung dieser vorbekannten Verbindungen gegenüber wichtigen Schadpflanzen ebenso wie ihre Verträglichkeit gegenüber Kulturpflanzen ist jedoch nicht immer in allen Anwendungsbereichen völlig zufriedenstellend.

Es wurden neue 2,4-Bis-(alkoximinoalkyl)-cyclohexan-1,3-dione der allgemeinen Formel (I),

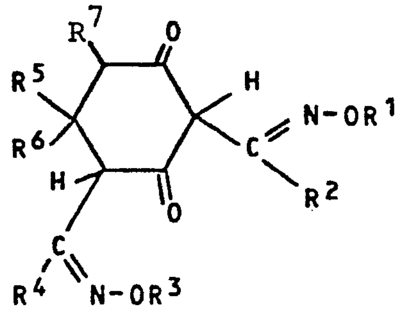

(I)

in welcher

$R^1$ und $R^3$ unabhängig voneinander jeweils für Wasserstoff, Alkyl, Alkenyl, Alkinyl, Alkoxyalkyl, Alkylthioalkyl, Halogenalkyl, Halogenalkenyl, Alkoxycarbonylalkyl, Alkoximinoalkyl, für jeweils gegebenenfalls substituiertes Aralkyl, Aryl oder Heteroarylalkyl oder für Tetrahydropyranyl stehen,

$R^2$ und $R^4$ unabhängig voneinander jeweils für Wasserstoff, Alkyl, Alkenyl, Alkinyl, Alkoxyalkyl, Alkylthioalkyl, Halogenalkyl, Cycloalkyl oder für jeweils gegebenenfalls substituiertes Aryl, Aryloxyalkyl oder Arylthioalkyl stehen,

$R^5$ und $R^6$ unabhängig voneinander jeweils für Wasserstoff, Alkyl, Alkenyl, Alkoxyalkyl, Alkylthioalkyl oder für jeweils gegebenenfalls substituiertes Cycloalkyl, Cycloalkenyl, Aryl, Heteroaryl, Aralkyl, Aryloxyalkyl oder Arylthioalkyl stehen und

$R^7$ für Wasserstoff steht oder gemeinsam mit $R^6$ für einen zweifach verknüpften Alkandiylrest steht,

sowie deren Metallsalze gefunden.

Die Verbindungen der Formel (I) können als Gemische der Tautomeren (Ia) - (If) vorliegen:

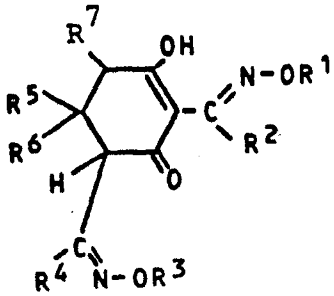

(Ia)

;

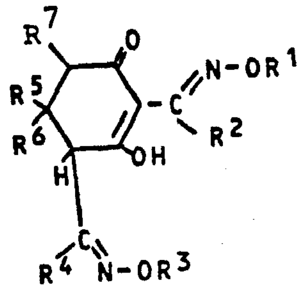

(Ib)

;

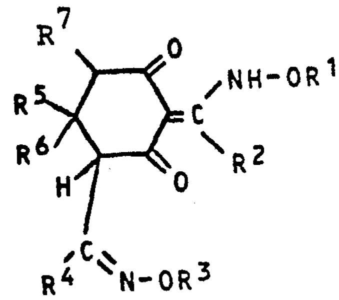

(Ic)

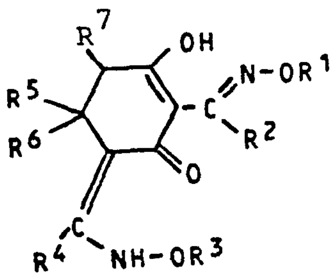

(Id)

;

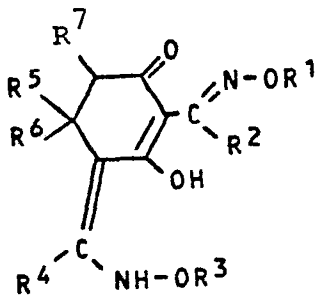

(Ie)

;

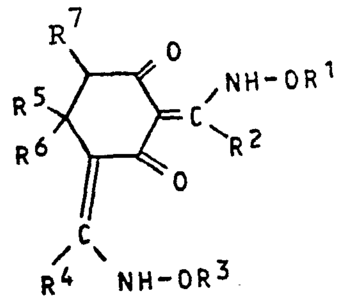

(If)

in welcher

$R^1$, $R^2$, $R^3$, $R^4$, $R^5$, $R^6$ und $R^7$ die oben angegebene Bedeutung haben,

die durch starke intramolekulare Wasserstoffbrückenbindungen stabilisiert werden.

Außerdem können die Verbindungen der Formel (I) als geometrische und/oder optische Isomere oder Isomerengemische unterschiedlicher Zusammensetzung vorliegen. Sowohl die reinen Isomeren als auch die Isomerengemische, ebenso wie die verschiedenen tautomeren Strukturen werden erfindungsgemäß beansprucht.

Weiterhin wurde gefunden, daß man die neuen 2,4-Bis-(alkoximinoalkyl)-cyclohexan-1,3-dione der allgemeinen Formel (I),

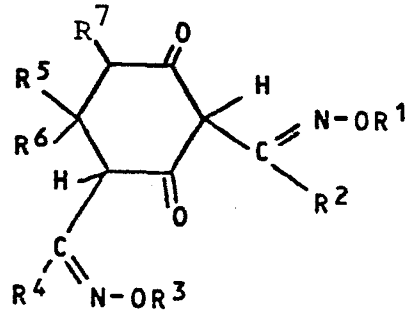

(I)

in welcher

$R^1$ und $R^3$ unabhängig voneinander jeweils für Wasserstoff, Alkyl, Alkenyl, Alkinyl, Alkoxyalkyl, Alkylthioalkyl, Halogenalkyl, Halogenalkenyl, Alkoxycarbonylalkyl, Alkoximinoalkyl, für jeweils gegebenenfalls substituiertes Aralkyl, Aryl oder Heteroarylalkyl oder für Tetrahydropyranyl stehen,

$R^2$ und $R^4$ unabhängig voneinander jeweils für Wasserstoff, Alkyl, Alkenyl, Alkinyl, Alkoxyalkyl, Alkylthioalkyl, Halogenalkyl, Cycloalkyl oder für jeweils gegebenenfalls substituiertes Aryl, Aryloxyalkyl oder Arylthioalkyl stehen und

$R^5$ und $R^6$ unabhängig voneinander jeweils für Wasserstoff, Alkyl, Alkenyl, Alkoxyalkyl, Alkylthioalkyl oder für jeweils gegebenenfalls substituiertes Cycloalkyl, Cycloalkenyl, Aryl, Heteroaryl, Aralkyl, Aryloxyalkyl oder Arylthioalkyl stehen und

$R^7$ für Wasserstoff steht oder gemeinsam mit $R^6$ für einen zweifach verknüpften Alkandiylrest steht,

erhält, wenn man

(a) Ketone der Formel (II),

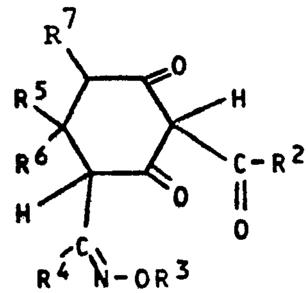

(II)

in welcher

$R^2$, $R^3$, $R^4$, $R^5$, $R^6$ und $R^7$ die oben angegebene Bedeutung haben,

mit Hydroxylamin-Derivaten der Formel (III),

$$H_2N - O - R^1 \qquad (III)$$

in welcher

$R^1$ die oben angegebene Bedeutung hat,

oder mit deren Hydrosalzen, gegebenenfalls in Gegenwart eines Verdünnungsmittels und gegebenenfalls in Gegenwart eines Säurebindemittels umsetzt, oder wenn man

(b) die nach Verfahren (a) erhältlichen Oxime der Formel (Ia),

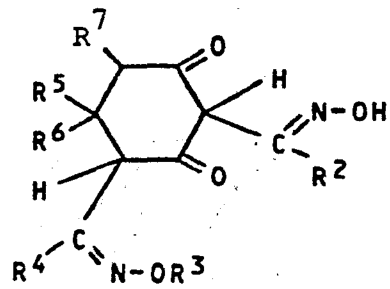

(Ia)

in welcher

$R^2$, $R^3$, $R^4$, $R^5$, $R^6$ und $R^7$ die oben angegebene Bedeutung haben,

mit Alkylierungsmitteln der Formel (IV),

$$R^{1'} - X \quad \text{(IV)}$$

in welcher

$R^{1'}$ für die gleichen Reste wie $R^1$ steht, mit Ausnahme des Wasserstoffrestes, und

X für eine elektronenanziehende Abgangsgruppe steht,

gegebenenfalls in Gegenwart eines Verdünnungsmittels sowie gegebenenfalls in Gegenwart eines Säurebindemittels und gegebenenfalls in Gegenwart eines Katalysators umsetzt.

Schließlich wurde gefunden, daß die neuen 2,4-Bis-(alkoximinoalkyl)-cyclohexan-1,3-dione der Formel (I) sowie deren Metallsalze herbizide, insbesondere auch selektiv-herbizide Eigenschaften besitzen.

Überraschenderweise besitzen die neuen 2,4-Bis-(alkoximinoalkyl)-cyclohexan-1,3-dione der Formel (I) sowie deren Metallsalze eine erheblich bessere herbizide Wirksamkeit als die aus dem Stand der Technik bekannten Cyclohexan-1,3-dione, wie beispielsweise das 2-(Allyloximino)-butyl-5,5-dimethyl-4-methoxycarbonyl-cyclohexan-1,3-dion-Mononatriumsalz, welche chemisch und wirkungsmäßig naheliegende Verbindungen sind.

Die erfindungsgemäßen 2,4-Bis-(alkoximinoalkyl)-cyclohexan-1,3-dione sind durch die Formel (I) allgemein definiert.

Bevorzugt sind Verbindungen der Formel (I), bei welchen

$R^1$ und $R^3$ unabhängig voneinander jeweils für Wasserstoff, für jeweils geradkettiges oder verzweigtes Alkyl, Alkenyl, Alkinyl, Alkoxyalkyl, Alkylthioalkyl, Halogenalkyl oder Halogenalkenyl mit jeweils bis zu 12 Kohlenstoffatomen in den einzelnen Alkyl- bzw. Alkenyl- oder Alkinylteilen und gegebenenfalls bis zu 9 gleichen oder verschiedenen Halogenatomen stehen, außerdem für jeweils geradkettiges oder verzweigtes Alkoxycarbonylalkyl oder Alkoximinoalkyl mit jeweils bis zu 4 Kohlenstoffatomen in den entsprechenden Alkylteilen oder für jeweils gegebenenfalls einfach oder mehrfach, gleich oder verschieden substituiertes Phenyl oder Phenylalkyl mit gegebenenfalls bis zu 4 Kohlenstoffatomen im geradkettigen oder verzweigten Alkylteil stehen, wobei als Phenylsubstituenten infrage kommen: Halogen, Cyano, Nitro sowie jeweils geradkettiges oder verzweigtes Alkyl, Alkoxy, Alkylthio, Halogenalkyl, Halogenalkoxy, Halogenalkylthio, Dioxyalkylen oder Phenylalkoxy mit jeweils bis zu 4 Kohlenstoffatomen in den einzelnen Alkylteilen und gegebenenfalls bis zu 9 gleichen oder verschiedenen Halogenatomen; ferner für einen gegebenenfalls einfach oder mehrfach, gleich oder verschieden durch Halogen, niederes Alkyl oder niederes Halogenalkyl substituierten 5-gliedrigen oder 6-gliedrigen, über eine Alkylenbrücke mit 1 bis 4 Kohlenstoffatomen verknüpften Heterocyclus mit 1 bis 3 gleichen oder verschiedenen Heteroatomen stehen, wobei als Heteroatome infrage kommen: Stickstoff, Sauerstoff oder Schwefel, und außerdem für Tetrahydropyranyl stehen,

$R^2$ und $R^4$ unabhängig voneinander jeweils für Wasserstoff, für jeweils geradkettiges oder verzweigtes Alkyl, Alkenyl, Alkinyl, Alkoxyalkyl, Alkylthioalkyl oder Halogenalkyl mit jeweils bis zu 12 Kohlenstoffatomen in den einzelnen Alkyl- bzw. Alkenyl- oder Alkinylteilen und mit gegebenenfalls bis zu 9 gleichen oder verschiedenen Halogenatomen, für Cycloalkyl mit 3 bis 7 Kohlenstoffatomen oder für jeweils gegebenenfalls einfach oder mehrfach, gleich oder verschieden substituiertes Phenyl, Phenoxyalkyl oder Phenylthioalkyl mit gegebenenfalls bis zu 4 Kohlenstoffatomen in den geradkettigen oder verzweigten Alkylteilen stehen, wobei als Phenylsubstituenten die bei $R^1$ und $R^3$ genannten infrage kommen,

$R^5$ und $R^6$ unabhängig voneinander jeweils für Wasserstoff, für jeweils geradkettiges oder verzweigtes Alkyl, Alkenyl, Alkoxyalkyl oder Alkylthioalkyl mit bis zu 8 Kohlenstoffatomen in den einzelnen Alkylteilen, für jeweils gegebenenfalls einfach oder mehrfach, gleich oder verschieden durch Halogen oder $C_1$-$C_4$-Alkyl substituiertes Cycloalkyl oder Cycloalkenyl mit jeweils 3 bis 7 Kohlenstoffatomen oder für jeweils gegebenenfalls einfach oder mehrfach, gleich oder verschieden substituiertes Phenyl, Phenylalkyl, Phenoxyalkyl oder Phenylthioalkyl mit gegebenenfalls 1 bis 4 Kohlenstoffatomen in den Alkylteilen stehen, wobei als Substituenten die bei $R^1$ genannten infrage kommen oder für einen gegebenenfalls einfach oder mehrfach, gleich oder verschieden substituierten 5-gliedrigen oder 6-gliedrigen Heterocyclus mit 1 bis 3 Heteroatomen stehen, wobei als Heteroatome Stickstoff, Sauerstoff oder Schwefel genannt seien und wobei als Substituenten infrage kommen: Halogen, Cyano, Nitro sowie jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen und

$R^7$ für Wasserstoff steht oder gemeinsam mit $R^6$ für einen geradkettigen oder verzweigten zweifach verknüpften Alkandiylrest mit 3 bis 12 Kohlenstoffatomen steht,

sowie deren Metallsalze.

Besonders bevorzugt sind Verbindungen der Formel (I), bei welchen

$R^1$ und $R^3$ unabhängig voneinander jeweils für Wasserstoff, Methyl, Ethyl, n- oder i-Propyl, n-, i-, s- oder t-Butyl, n- oder i-Pentyl, n- oder i-Hexyl, Undecyl, Allyl, n- oder i-Butenyl, n- oder i-Pentenyl, Propargyl, Methoxymethyl, Ethoxymethyl, Isopropoxymethyl, Methoxyethyl, Ethoxyethyl, Methylthiomethyl, Methylthioethyl, Ethylthioethyl, 2-Chlorethyl, 2-Bromethyl, 2,2,2-Trifluorethyl, 2,2,2-Trichlorethyl, 3-Chlorpropenyl, Methoxycarbonylmethyl, Ethoxycarbonylmethyl, Methoximinomethyl, Methoximinoethyl, Methoximino-2-propyl, jeweils gegebenenfalls ein- bis dreifach, gleich oder verschieden durch Fluor, Chlor, Brom, Cyano, Nitro, Methyl, Methoxy oder Trifluormethyl substituiertes Phenyl oder Benzyl, für einen gegebenenfalls ein- bis dreifach, gleich oder verschieden durch Chlor, Methyl oder Trifluormethyl substituierten, über eine Methylenbrücke verknüpften Heterocyclus der Formel

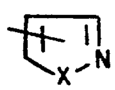 ; 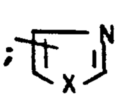 ; 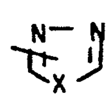 oder 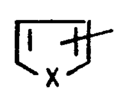

stehen, wobei X jeweils für Sauerstoff oder Schwefel steht, oder für Tetrahydropyranyl stehen,

$R^2$ und $R^4$ unabhängig voneinander jeweils für Wasserstoff, Methyl, Ethyl, n- oder i-Propyl, n-, i-, s- oder t-Butyl, n- oder i-Pentyl, n- oder i-Hexyl, Undecyl, Vinyl, Allyl, n- oder i-Butenyl, n- oder i-Pentenyl, n- oder i-Hexenyl, Propargyl, Methylpropargyl, Dimethylpropargyl, Methoxymethyl, Ethoxymethyl, Isopropoxymethyl, Methoxyethyl, Ethoxyethyl, Methylthiomethyl, Methylthioethyl, Ethylthioethyl, 2-Ethylthio-1-propyl, Trifluormethyl, Dichlorfluormethyl, Difluorchlormethyl, Trichlormethyl, Dichlormethyl, Chlormethyl, 2-Chlorethyl, 2-Bromethyl, 2,2,2-Trichlorethyl, 2,2,2-Trifluorethyl, Cyclopropyl, Cyclobutyl, Cyclopentyl, Cyclohexyl oder jeweils gegebenenfalls ein- bis dreifach, gleich oder verschieden durch Fluor, Chlor, Brom, Cyano, Nitro, Methyl, Methoxy oder Trifluormethyl substituiertes Phenyl, Phenoxymethyl oder Phenylthiomethyl stehen,

$R^5$ und $R^6$ unabhängig voneinander jeweils für Wasserstoff, Methyl, Ethyl, n- oder i-Propyl, n-, i-, s- oder t-Butyl, Vinyl, Allyl, n- oder i-Butenyl, n- oder i-Pentenyl, n- oder i-Hexenyl, Methoxymethyl, Ethoxymethyl, Methoxyethyl, Ethoxyethyl, Methylthiomethyl, Ethylthiomethyl, Ethylthioethyl, Ethylthiopropyl, oder für jeweils gegebenenfalls ein- bis dreifach, gleich oder verschieden durch Fluor, Chlor, Brom, Cyano, Nitro, Methyl, Methoxy, Ethoxy, Dioxymethylen, Benzyloxy, Methylthio, Ethylthio oder Trifluormethyl substituiertes Phenyl, Benzyl, Phenylethyl, Phenoxymethyl, Phenoxyethyl,

Phenylthiomethyl, Phenylthioethyl oder Phenylthiopropyl stehen oder für jeweils gegebenenfalls ein- bis dreifach, gleich oder verschieden durch Fluor, Chlor, Brom, Methyl oder Ethyl substituiertes Cyclopropyl, Cyclopentyl, Cyclohexyl, Cyclohexenyl oder Bicyclo-[2,2,1]-heptenyl oder für einen gegebenenfalls ein- bis dreifach, gleich oder verschieden durch Chlor, Methyl oder Trifluormethyl substituierten Heterocyclus der Formel

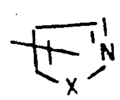 ; 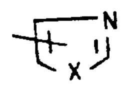 ; 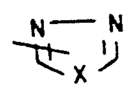 oder 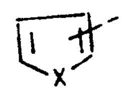

stehen, wobei X jeweils für Sauerstoff oder Schwefel steht und

$R^7$ für Wasserstoff steht, oder gemeinsam mit $R^6$ für 1,3-Propandiyl, 1,4 Butandiyl, 1,5-Pentandiyl oder 1,6-Hexandiyl steht.

Im einzelnen seien außer den bei den Herstellungsbeispielen genannten Verbindungen die folgenden 2,4-Bis-(alkoximinoalkyl)-cyclohexan-1,3-dione der allgemeinen Formel (I) genannt:

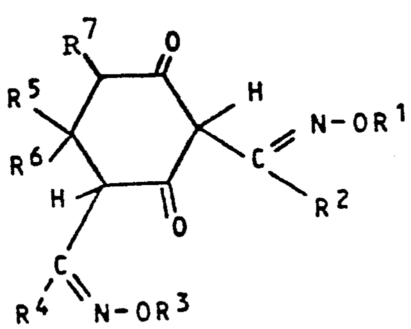

(I)

Tabelle 1:

| $R^1$ | $R^2$ | $R^3$ | $R^4$ | $R^5$ | $R^6$ | $R^7$ |
|---|---|---|---|---|---|---|
| $CH_3$ | $CH_3(CH_2)_2-$ | $CH_3$ | $CH_3$ | $C_2H_5S\overset{CH_3}{\overset{\vert}{C}}HCH_2-$ | H | H |
| $CH_3$ | $CH_3(CH_2)_2-$ | $CH_3$ | $CH_3$ | $C_2H_5\overset{CH_3}{\overset{\vert}{C}}HCH_2-$ | $CH_3$ | H |
| $CH_2=CH-CH_2-$ | $CH_3(CH_2)_2-$ | $CH_3$ | $CH_3$ | $C_2H_5S\overset{CH_3}{\overset{\vert}{C}}HCH_2-$ | H | H |
| $CH_2=CH-CH_2-$ | $CH_3(CH_2)_2-$ | $CH_3$ | $CH_3$ | $C_2H_5S\overset{CH_3}{\overset{\vert}{C}}HCH_2-$ | $CH_3$ | H |
| $CH_2=CH-CH_2-$ | $CH_3$ | $CH_3$ | $CH_3$ | $C_2H_5S\overset{CH_3}{\overset{\vert}{C}}HCH_2-$ | $CH_3$ | H |
| $Cl-CH=CH-CH_2-$ | $CH_3$ | $CH_3$ | $CH_3$ | $CH_3$ | $CH_3$ | H |
| $Cl-CH=CH-CH_2-$ | $CH_3(CH_2)_2-$ | $CH_3$ | $CH_3$ | $CH_3$ | $CH_3$ | H |

Tabelle 1: (Fortsetzung)

| $R^1$ | $R^2$ | $R^3$ | $R^4$ | $R^5$ | $R^6$ | $R^7$ |
|---|---|---|---|---|---|---|
| $CH_3$<br>\|<br>$CH_3ON=CH-CH$ | $CH_3$ | $CH_3$ | $CH_3$ | $CH_3$ | $CH_3$ | H |
| $CH_3$<br>\|<br>$CH_3ON=CH-CH-$ | $CH_3(CH_2)_2-$ | $CH_3$ | $CH_3$ | $CH_3$ | $CH_3$ | H |
| $CH_2=CH-CH_2-$ | $CH_3(CH_2)_2-$ | $CH_3$ | $CH_3$ | $C_6H_5-$ | H | H |
| $CH_3$ | $CH_3$ | $CH_3$ | $CH_3$ | $C_6H_5-$ | H | H |
| $CH_2=CH-CH_2-$ | $CH_3(CH_2)_2$ | $-CH_2-$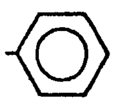 | $CH_3$ | $CH_3$ | $CH_3$ | H |
| $CH_2=CH-CH_2-$ | $CH_3(CH_2)_2-$ | $CH_3$ | $CH_3$ | H | H | H |
| $C_2H_5-$ | $CH_3(CH_2)_2-$ | $CH_3$ | $C_3H_7$ | H | H | H |
| $C_2H_5-$ | $CH_3(CH_2)_2-$ | $CH_3$ | 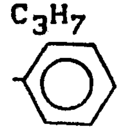 | H | H | H |
| $CH_2=CH-CH_2-$ | $CH_3(CH_2)_2-$ | $CH_3$ | $C_3H_7$ | $CH_3$ | H | H |
| $C_2H_5-$ | 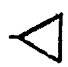 | $CH_3$ | $CH_3$ | $CH_3$ | $CH_3$ | H |

Tabelle 1: (Fortsetzung)

| $R^1$ | $R^2$ | $R^3$ | $R^4$ | $R^5$ | $R^6$ | $R^7$ |
|---|---|---|---|---|---|---|
| $C_2H_5$- | 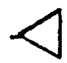 | $CH_2$=CH-$CH_2$- | $CH_3$ | $CH_3$ | $CH_3$ | H |
| $CH_2$=CH-$CH_2$- | 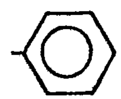 | $CH_3$ | $CH_3$ | $CH_3$ | $CH_3$ | H |
| $CH_2$=CH-$CH_2$- | $CH_3(CH_2)_2$- | $CH_2$=CH-$CH_2$- | $CH_3$ | H | $-(CH_2)_4-$ | |
| $CH_2$=CH-$CH_2$- | $CH_3(CH_2)_2$- | $CH_3$ | $CH_3$ | H | $-(CH_2)_3-$ | |
| $CH_2$=CH-$CH_2$- | $CH_3(CH_2)_2$- | $CH_3$ | $CH_3CH(CH_3)CH_2$- | $CH_3$ | $CH_3$ | H |
| $CH_2$=CH-$CH_2$- | $CH_3(CH_2)_2$- | $CH_3$ | $CH_3$ | 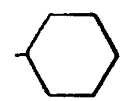 | $CH_3$ | H |
| $CH_2$=CH-$CH_2$- | $CH_3(CH_2)_2$- | $CH_3$ | $CH_3$ | 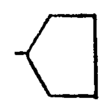 | H | H |
| $C_2H_5$- | $C_2H_5$- | $CH_3$ | 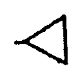 | $CH_3$ | $CH_3$ | H |
| $C_2H_5$- | $C_2H_5$- | n-$C_4H_9$- | H | $CH_3$ | $CH_3$ | H |

0172551

Tabelle 1: (Fortsetzung)

| $R^1$ | $R^2$ | $R^3$ | $R^4$ | $R^5$ | $R^6$ | $R^7$ |
|---|---|---|---|---|---|---|
| $C_2H_5-$ | $CH_3(CH_2)_2-$ | $-C_2H_5$ | $CH_3(CH_2)_2-$ | $CH_3OCH_2-$ | H | H |
| $C_2H_5-$ | $CH_3(CH_2)_2-$ | $-C_2H_5$ | $CH_3$ | 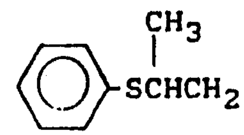 | H | H |
| $CH_2=CHCH_2-$ | $CH_3(CH_2)_2-$ | $CH_3$ | $C_3H_7$ | 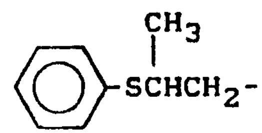 | H | H |
| $CH_2=CHCH_2-$ | $CH_3CH_2-$ | $CH_3$ | H | 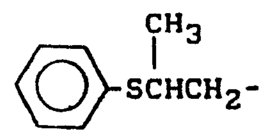 | H | H |

Verwendet man beispielsweise 2-Acetyl-5,5-dimethyl-4-(1-methoximino-ethyl)-cyclohexan-1,3-dion und O-Allylhydroxylamin als Ausgangsstoffe, so läßt sich der Reaktionsablauf des erfindungsgemäßen Verfahrens (a) durch das folgende Formelschema darstellen:

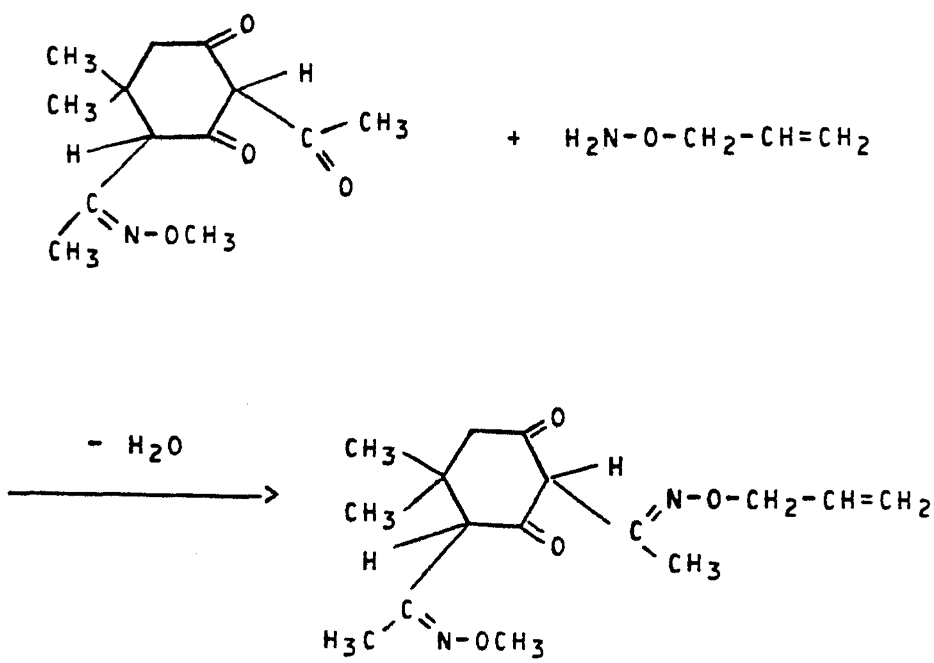


Verwendet man beispielsweise 5,5-Dimethyl-2-(1-hydroximinobutyl)-4-(1-methoximino-ethyl)-cyclohexan-1,3-dion und Methyliodid als Ausgangsstoffe, so läßt sich der Reaktionsablauf des erfindungsgemäßen Verfahrens (b) durch das folgende Formelschema darstellen:

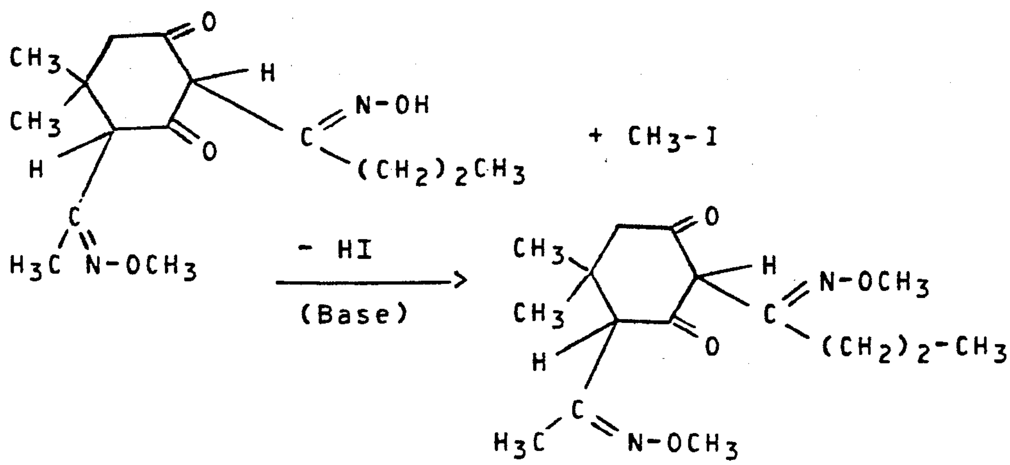


Die zur Durchführung des erfindungsgemäßen Verfahrens (a) als Ausgangsstoffe benötigten Ketone sind durch die Formel (II) allgemein definiert. In dieser Formel (II) stehen $R^2$, $R^3$, $R^4$, $R^5$, $R^6$ und $R^7$ vorzugsweise für diejenigen Reste, die schon bei der Beschreibung der erfindungsgemäßen Stoffe der Formel (I) als bevorzugt für diese Substituenten genannt wurden.

Die Ketone der Formel (II) sind noch nicht bekannt. Man erhält sie, wenn man ß-Oximino-carbonsäureester der Formel (V),

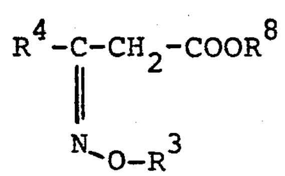

(V)

in welcher

$R^3$ und $R^4$ die oben angegebene Bedeutung haben und

$R^8$ für geradkettiges oder verzweigtes Alkyl, insbesondere für Methyl oder Ethyl steht,

zunächst in einer ersten Stufe mit Methyl-vinyl-ketonen der Formel (VI),

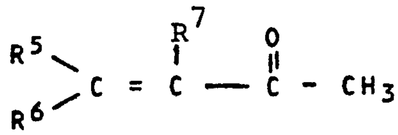

(VI)

in welcher

$R^5$, $R^6$ und $R^7$ die oben angegebene Bedeutung haben,

gegebenenfalls in Gegenwart eines Verdünnungsmittels (wie beispielsweise Ethanol) und gegebenenfalls in Gegenwart einer Base (wie beispielsweise Natriumethylat) bei Temperaturen zwischen +50°C und +200°C cyclisiert und die so erhältlichen Cyclohexan-1,3-dione der Formel (VII),

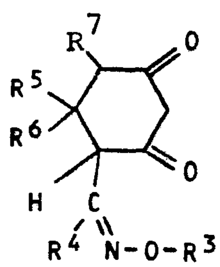

(VII)

in welcher

$R^3$, $R^4$, $R^5$, $R^6$ und $R^7$ die oben angegebene Bedeutung haben,

entweder zunächst in einer zweiten Stufe mit Acylierungsmitteln der Formel (VIII),

$$R^2 - \overset{\overset{\displaystyle O}{\|}}{C} - X' \qquad \text{(VIII)}$$

in welcher

$R^2$ die oben angegebene Bedeutung hat und

X' für eine elektronenanziehende Abgangsgruppe, insbesondere für Halogen, Cyano oder den Rest $R^2-\underset{\underset{\displaystyle O}{\|}}{C}-O-$ steht, wobei $R^2$ die oben angegebene Bedeutung hat,

gegebenenfalls in Gegenwart eines Verdünnungsmittels (wie beispielsweise Toluol) und gegebenenfalls in Gegenwart eines Säurebindemittels (wie beispielsweise Diazabicycloundecen (DBU)) bei Temperaturen zwischen -20°C und +50°C umsetzt und die so erhältlichen O-Acylderivate der Formel (IX),

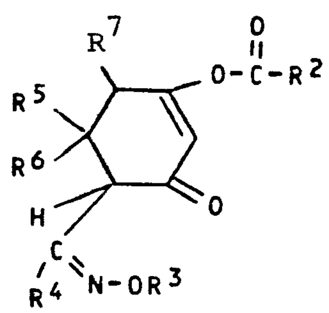

(IX)

in welcher

$R^2$, $R^3$, $R^4$, $R^5$, $R^6$ und $R^7$ die oben angegebene Bedeutung haben,

in einer 3.Stufe, ebenfalls gegebenenfalls in Gegenwart eines Verdünnungsmittels (wie beispielsweise Toluol), und in Gegenwart eines basischen Katalysators (wie beispielsweise 4-(N,N-Dimethylamino)-pyridin) bei Temperaturen zwischen 0°C und +120°C umlagert,

oder wenn man die 2. und die 3.Stufe in einem Reaktionsschritt ebenfalls in Gegenwart eines Verdünnungsmittels (wie beispielsweise Pyridin oder Dichlormethan), gegebenenfalls in Gegenwart eines säurebindenden Mittels (wie beispielsweise Triethylamin) und gegebenenfalls in Gegenwart eines Katalysators (wie beispielsweise Zinkchlorid) bei Temperaturen zwischen 0°C und +150°C durchführt.

Die ß-Oximinocarbonsäureester der Formel (V) sind bekannt (vergleiche z. B. Zh. Org. Khim. 9, 1133-1137 /1973/; C. A. 79, 65531z) oder lassen sich nach prinzipiell bekannten Verfahren in analoger Weise herstellen.

Die Methyl-vinyl-ketone der Formel (VI) und die Acylierungsmittel der Formel (VIII) sind allgemein bekannte Verbindungen der organischen Chemie.

Die Cyclohexan-1,3-dione der Formel (VII) sind noch nicht bekannt. Man erhält sie durch Isolierung bei der Herstellung der Ketone der Formel (II).

Die weiterhin zur Durchführung des erfindungsgemäßen Verfahrens (a) als Ausgangsstoffe benötigten Hydroxylamin-Derivate sind durch die Formel (III) allgemein definiert. In dieser Formel (III) steht $R^2$ vorzugsweise für diejenigen Substituenten, die schon bei der Beschreibung der erfindungsgemäßen Stoffe der Formel (I) als bevorzugt für diese Reste genannt wurden.

Die Hydroxylamin-Derivate der Formel (III) und deren Hydrosalze sind ebenfalls allgemein bekannte Verbindungen der organischen Chemie.

Die zur Durchführung des erfindungsgemäßen Verfahrens (b) als Ausgangsstoffe benötigten Oxime sind durch die Formel (Ia) allgemein definiert. In dieser Formel (Ia) stehen $R^2$, $R^3$, $R^4$, $R^5$, $R^6$ und $R^7$ vorzugsweise für diejenigen Reste, die bereits bei der Beschreibung der erfindungsgemäßen Stoffe der Formel (I) als bevorzugt für diese Substituenten genannt wurden. Die Oxime der Formel (Ia) sind erfindungsgemäße Verbindungen und erhältlich mit Hilfe des erfindungsgemäßen Verfahrens (a).

Die zur Durchführung des erfindungsgemäßen Verfahrens (b) weiterhin als Ausgangsstoffe benötigten Alkylierungsmittel sind durch die Formel (IV) allgemein definiert.

In dieser Formel (IV) steht $R^{1'}$ vorzugsweise für diejenigen Reste, die schon bei der Beschreibung der erfindungsgemäßen Stoffe der Formel (I) als bevorzugt für den Substituenten $R^1$ genannt wurden, mit Ausnahme des Wasserstoffrestes. X steht vorzugsweise für Halogen oder für jeweils gegebenenfalls substituiertes Alkylsulfonyloxy, Alkoxysulfonyloxy oder Arylsulfonyloxy, insbesondere für Chlor, Brom, Iod, Methansulfonyloxy, Methoxysulfonyloxy oder p-Toluolsulfonyloxy.

Die Alkylierungsmittel der Formel (IV) sind allgemein bekannte Verbindungen der organischen Chemie.

Als Verdünnungsmittel zur Durchführung des erfindungsgemäßen Verfahrens (a) kommen inerte organische Lösungsmittel infrage. Vorzugsweise verwendet man Alkohole oder wäßrige Alkoholgemische, beispielsweise Methanol, Ethanol, n- und i-Propanol, sowie deren Gemische mit Wasser.

Werden die Hydroxylamin-Derivate der Formel (III) in Form ihrer Salze - vorzugsweise verwendet man die Hydrochloride - eingesetzt, so arbeitet man üblicherweise in Gegenwart eines Säurebindemittels.

Als solche kommen alle üblichen anorganischen oder organischen Basen infrage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, Alkalimetallacetate, wie Natriumacetat oder Alkalimetallalkoholate, wie Natrium- oder Kaliummethylat oder -ethylat.

Die Reaktionstemperaturen können bei der Durchführung des erfindungsgemäßen Verfahrens (a) in einem größeren Bereich variiert werden. Im allgemeinen arbeitet man zwischen 0°C und +150°C, vorzugsweise zwischen +20°C und +100°C.

Bei der Durchführung des erfindungsgemäßen Verfahrens (a) setzt man pro Mol an Keton der Formel (II) im allgemeinen 1,0 bis 2,0 Mol, vorzugsweise 1,0 bis 1,5 Mol an Hydroxylamin-Derivat der Formel (III) und gegebenenfalls 1,0 bis 2.0 Mol, vorzugsweise 1,0 bis 1,5 Mol an Säurebindemittel ein. Die Aufarbeitung und Isolierung der Reaktionsprodukte der Formel (I) erfolgt nach üblichen Methoden.

Als Verdünnungsmittel zur Durchführung des erfindungsgemäßen Verfahrens (b) kommen ebenfalls inerte organische Lösungsmittel infrage.

Hierzu gehören insbesondere Ketone, wie Aceton oder Butanon, Nitrile, wie Acetonitril oder Propionitril, Ether wie Diethylether, Dioxan oder Tetrahydrofuran, aliphatische oder aromatische, gegebenenfalls halogenierte Kohlenwasserstoffe, wie beispielsweise Petrolether, Benzol, Toluol, Xylol, Chlorbenzol, Dichlormethan, Chloroform oder Tetrachlorkohlenstoff, sowie Ester, wie beispielsweise Essigsäureethylester, oder Amide, wie Dimethylformamid oder Hexamethylphosphorsäuretriamid.

Das erfindungsgemäße Verfahren (b) kann auch in einem

organisch-wäßrigen Zweiphasensystem wie beispielsweise Dichlormethan/Wasser oder Toluol/Wasser durchgeführt werden.

Das erfindungsgemäße Verfahren (b) wird gegebenenfalls in Gegenwart eines Säurebindemittels durchgeführt. Als solche kommen alle üblicherweise verwendbaren organischen und anorganischen Basen infrage. Insbesondere genannt seien Alkalimetallhydride oder -amide, wie Natriumhydrid oder Natriumamid oder Alkalimetallalkoholate, wie Natriummethylat oder Natriumethylat.

Die Reaktionstemperaturen können bei dem erfindungsgemäßen Verfahren (b) in einem größeren Bereich variiert werden. Im allgemeinen arbeitet man zwischen -20°C und +100°C, vorzugsweise zwischen 0°C und +80°C.

Bei der Durchführung des erfindungsgemäßen Verfahrens (b) setzt man pro Mol Oxim der Formel (Ia) im allgemeinen 1 bis 3 Mol, insbesondere 1 bis 1,5 Mol an Alkylierungsmittel der Formel (IV) und 1 bis 10 Mol, vorzugsweise 1 bis 2 Mol an Säurebindemittel ein.

Führt man die Umsetzung in einem organisch-wäßrigen Zweiphasensystem durch, kann man gegebenenfalls in Gegenwart von 0,1 bis 1 Mol eines geeigneten Phasentransferkatalysators, wie beispielsweise einer quartären Ammonium- oder Phosphoniumverbindung, arbeiten. Beispielhaft seien Triethylbenzylammoniumchlorid und Benzyl-dodecyl-dimethylammoniumchlorid genannt.

Die Aufarbeitung und Isolierung der Reaktionsprodukte der Formel (I) erfolgt nach üblichen Methoden.

Zur Herstellung von Metallsalzen der Verbindungen der Formel (I) kommen vorzugsweise Salze von Metallen der I. bis IV:Haupt- und der I. und II. sowie der IV. bis VIII. Nebengruppe des Periodensystems infrage, wobei Natrium, Kalium, Kupfer, Zink, Mangan, Magnesium, Calcium, Barium, Zinn, Eisen, Cobalt und Nickel beispielhaft genannt seien.

Zur Herstellung der Natrium- und Kaliumsalze setzt man eine Verbindung der Formel (I) in wäßriger Lösung oder einem organischen Lösungsmittel, wie Aceton, Methanol, Ethanol oder Dimethylformamid, mit Natrium- bzw. Kaliumhydroxid um und isoliert die Salze durch Abfiltrieren oder durch Eindampfen der Lösung und reinigt sie gegebenenfalls durch Umkristallisieren.

Die Calcium-, Barium-, Magnesium-, Mangan-, Kupfer-, Nickel-, Zinn-, Eisen- und Cobaltsalze werden hergestellt aus den Natriumsalzen durch Behandeln mit einem entsprechenden anorganischen Metallsalz, z. B. Calciumchlorid, Bariumchlorid, Kupfersulfat, Nickelchlorid oder Cobaltnitrat. Die Calciumsalze können auch hergestellt werden durch Behandeln einer Verbindung der Formel (I) mit Calciumhydroxid.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die

an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei lassen sich die erfindungsgemäßen Wirkstoffe mit besonders gutem Erfolg zur Bekämpfung von monokotylen und dikotylen Unkräutern, einschließlich Ausfallgetreide und Ausfallmais in dikotylen Kulturen wie beispielsweise in Zuckerrüben, Soja, Raps oder Baumwolle einsetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoffimprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z. B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als

Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethyl-harnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage. Auch Mischung mit N,N-Dimethyl-N'-(3-trifluorphenyl)-harnstoff; 5-(2-Chlor-4-trifluormethylphenoxy)-2-nitrobenzoesäure; 5-(2-Chlor-4-trifluormethylphenoxy)-N-methylsulfonyl-2-nitrobenzamid; 3-Isopropyl-2,1,3-benzothiadiazin-4-on-2,2-dioxid; 5-Amino-4-chlor-2-phenyl-2,3-dihydro-3-oxo-pyridazin oder 3-(Methoxycarbonylaminophenyl)-N-(3'-methylphenyl)-carbamat sind möglich. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als

0172551

auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

In entsprechenden Aufwandmengen besitzen die erfindungsgemäßen Wirkstoffe (I) darüberhinaus auch eine fungizide Wirksamkeit und lassen sich beispielsweise zur Bekämpfung von Reiskrankheiten, wie z. B. gegen den Erreger der Reisfleckenkrankheit (Pyricularia oryzae) einsetzen.

Die Herstellung und die Verwendung der erfindungsgemäßen Wirkstoffe geht aus den nachfolgenden Beispielen hervor.

## Herstellungsbeispiele:

### Beispiel 1:

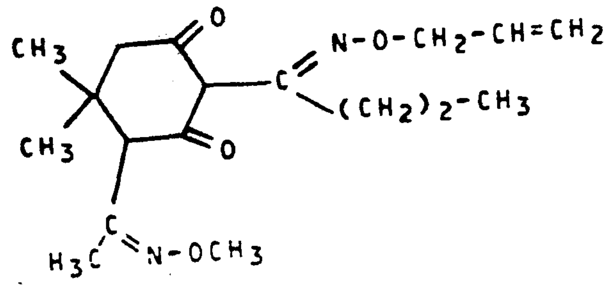

(Verfahren a)

60 g (0,217 Mol) 2-Butyryl-5,5-dimethyl-4-(1-methoximino-ethyl)-cyclohexan-1,3-dion in 200 ml Ethanol werden nacheinander mit 25,2 g (0,23 Mol) O-Allylhydroxylamin-Hydrochlorid in 50 ml Wasser und dann mit 115 ml 2-normaler Natronlauge versetzt und anschließend 16 Stunden bei Raumtemperatur gerührt. Zur Aufarbeitung entfernt man das Ethanol im Vakuum, extrahiert den Rückstand mehrfach mit Dichlormethan, wäscht die vereinigten organische Phasen mit verdünnter Salzsäure, trocknet über Magnesiumsulfat und entfernt das Lösungsmittel im Vakuum. Man erhält 65,3 g (97 % der Theorie) an 2-(1-Allyloximino-butyl)-5,5-dimethyl-4-(1-methoximino-ethyl)-cyclohexan-1,3-dion vom Brechungsindex $n_D^{24}$= 1,5072.

In entsprechender Weise und gemäß den allgemeinen Herstellungsangaben erhält man die folgenden 2,4-Bis-(alkoximinoalkyl)-cyclohexan-1,3-dione der allgemeinen Formel (I):

Tabelle 2:

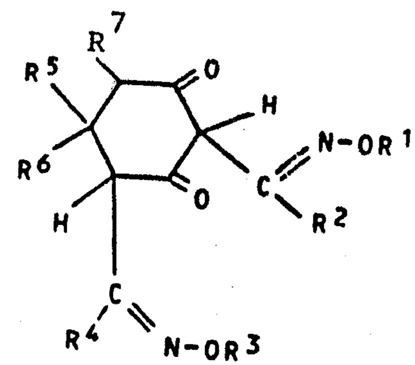

(I)

| Beispiel Nr. | $R^1$ | $R^2$ | $R^3$ | $R^4$ | $R^5$ | $R^6$ | $R^7$ | physikal. Daten |
|---|---|---|---|---|---|---|---|---|
| 2 | $CH_2=CHCH_2-$ | $n-C_3H_7$ | $CH_3$ | $CH_3$ | $C_6H_5$ | H | H | $n_D^{22.6}=$ 1.5411 |
| 3 | $CH_3$ | $n-C_3H_7$ | $CH_3$ | $CH_3$ | $CH_3$ | $CH_3$ | H | $n_D^{22.4}=$ 1.5098 |
| 4 | $C_2H_5$ | $n-C_3H_7$ | $CH_3$ | $CH_3$ | $C_6H_5$ | H | H | Öl |
| 5 | $C_2H_5$ | $n-C_3H_7$ | $CH_3$ | $CH_3$ | $CH_3$ | $CH_3$ | H | Öl |
| 6 | $CH_2=CHCH_2-$ | $n-C_3H_7$ | $CH_3$ | H | $CH_3$ | $CH_3$ | H | $n_D^{21.9}=$ 1.5209 |

**Tabelle 2:** (Fortsetzung)

| Bei-spiel Nr. | $R^1$ | $R^2$ | $R^3$ | $R^4$ | $R^5$ | $R^6$ | $R^7$ | physikal. Daten |
|---|---|---|---|---|---|---|---|---|
| 7 | $CH_3$ | n-$C_3H_7$ | $CH_3$ | $CH_3$ | 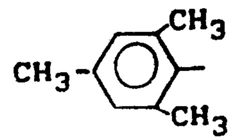 | H | H | Öl |
| 8 | $C_2H_5$ | n-$C_3H_7$ | $CH_3$ | $CH_3$ | 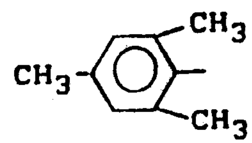 | H | H | Öl |
| 9 | $CH_2$=$CHCH_2$- | n-$C_3H_7$ | $CH_3$ | $CH_3$ | 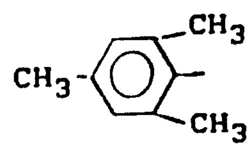 | H | H | Öl |
| 10 | 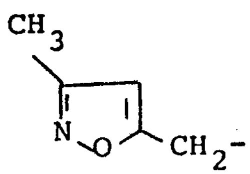 | n-$C_3H_7$ | $CH_3$ | $CH_3$ | 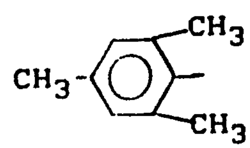 | H | H | Öl |

Tabelle 2: (Fortsetzung)

| Bei-spiel Nr. | $R^1$ | $R^2$ | $R^3$ | $R^4$ | $R^5$ | $R^6$ | $R^7$ | physikal. Daten |
|---|---|---|---|---|---|---|---|---|
| 11 | 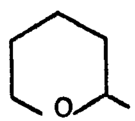 | $n-C_3H_7$ | $CH_3$ | $CH_3$ | 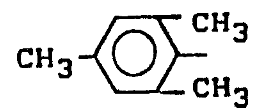 | H | H | Öl |
| 12 | $CH_2=CHCH_2-$ | $n-C_3H_7$ | $CH_3$ | $CH_3$ | 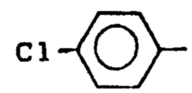 | H | H | $n_D^{22}$= 1,5563 |
| 13 | $C_2H_5$ | $n-C_3H_7$ | $CH_3$ | $CH_3$ | 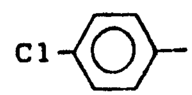 | H | H | $n_D^{21.2}$= 1.5560 |
| 14 | $CH_3$ | $C_2H_5$ | $CH_3$ | $CH_3$ | $C_6H_5$ | H | H | $n_D^{21}$ = 1.5471 |

Tabelle 2: (Fortsetzung)

| Beispiel Nr. | $R^1$ | $R^2$ | $R^3$ | $R^4$ | $R^5$ | $R^6$ | $R^7$ | physikal. Daten |
|---|---|---|---|---|---|---|---|---|
| 15 | $C_2H_5$ | $C_2H_5$ | $CH_3$ | $CH_3$ | $C_6H_5$ | H | H | $n_D^{22}$ = 1.5526 |
| 16 | $CH_2$=$CHCH_2$- | $CH_3$ | $CH_3$ | $CH_3$ | $C_6H_5$ | H | H | $n_D^{23.8}$= 1.5580 |
| 17 | 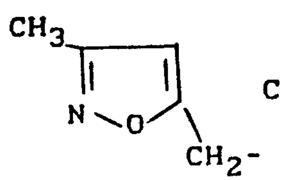 | $C_2H_5$ | $CH_3$ | $CH_3$ | $C_6H_5$ | H | H | $n_D^{21.2}$= 1.5459 |

Tabelle 2: (Fortsetzung)

| Beispiel Nr. | $R^1$ | $R^2$ | $R^3$ | $R^4$ | $R^5$ | $R^6$ | $R^7$ | physikal. Daten |
|---|---|---|---|---|---|---|---|---|
| 18 | 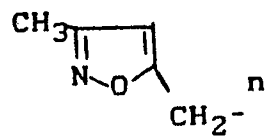 | n-$C_3H_7$ | $CH_3$ | $CH_3$ | 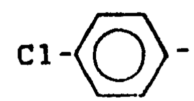 | H | H | $n_D^{21.1}$= 1.5412 |
| 19 | $CH_2$=$CHCH_2$- | $CH_3$ | $CH_3$ | $CH_3$ | $CH_3$ | $CH_3$ | H | $n_D^{22}$= 1.5235 |
| 20 | $CH_2$=$CHCH_2$- | $C_2H_5$ | $CH_3$ | $CH_3$ | $C_6H_5$ | H | H | $n_D^{22.3}$= 1.5585 |
| 21 | $C_2H_5$ | $CH_3$ | $CH_3$ | $CH_3$ | $C_6H_5$ | H | H | $n_D^{22.5}$= 1.5604 |

0172551

Tabelle 2: (Fortsetzung)

| Beispiel Nr. | $R^1$ | $R^2$ | $R^3$ | $R^4$ | $R^5$ | $R^6$ | $R^7$ | physikal. Daten |
|---|---|---|---|---|---|---|---|---|
| 22 | $C_2H_5$ | $CH_3$ | $CH_3$ | $CH_3$ | $CH_3$ | $CH_3$ | H | $n_D^{21}$ = 1.5228 |
| 23 | 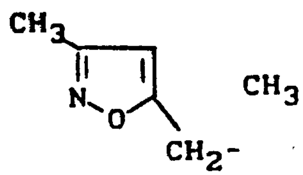 | $CH_3$ | $CH_3$ | $CH_3$ | $CH_3$ | $CH_3$ | H | $n_D^{21.2}$ = 1.5226 |
| 24 | 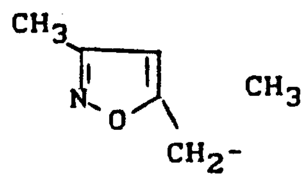 | $CH_3$ | $CH_3$ | $CH_3$ | $C_6H_5$ | H | H | $n_D^{21.2}$ = 1.5524 |
| 25 | $CH_3$ | $CH_3$ | $CH_3$ | $CH_3$ | $C_6H_5$ | H | H | $n_D^{21.1}$ = 1.5579 |

Tabelle 2: (Fortsetzung)

| Beispiel Nr. | $R^1$ | $R^2$ | $R^3$ | $R^4$ | $R^5$ | $R^6$ | $R^7$ | physikal. Daten |
|---|---|---|---|---|---|---|---|---|
| 26 | $CH_3$ | $CH_3$ | $CH_3$ | $CH_3$ | $CH_3$ | $CH_3$ | H | $n_D^{21.1}$ = 1.5234 |
| 27 | 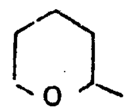 | $CH_3$ | $CH_3$ | $CH_3$ | $CH_3$ | $CH_3$ | H | $n_D^{21.2}$ = 1.5189 |
| 28 | 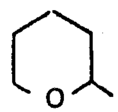 | $CH_3$ | $CH_3$ | $CH_3$ | $C_6H_5$ | H | H | $n_D^{21.2}$ = 1.5474 |
| 29 | $CH_3(CH_2)_3$- | n-$C_3H_7$ | $CH_3$ | $CH_3$ | $CH_3$ | $CH_3$ | H | $n_D^{22.1}$ = 1.4962 |
| 30 | $CH_3(CH_2)_3$- | n-$C_3H_7$ | $CH_3$ | $CH_3$ | $CH_3$ | $CH_3$ | H | $n_D^{22.1}$ = 1.5041 |

Tabelle 2: (Fortsetzung)

| Beispiel Nr. | $R^1$ | $R^2$ | $R^3$ | $R^4$ | $R^5$ | $R^6$ | $R^7$ | physikal. Daten |
|---|---|---|---|---|---|---|---|---|
| 31 | $C_2H_5OCOCH_2$- | n-$C_3H_7$ | $CH_3$ | $CH_3$ | $CH_3$ | $CH_3$ | H | $n_D^{22.7}$ = 1.4984 |
| 32 | $C_6H_5$-$CH_2$- | n-$C_3H_7$ | $CH_3$ | $CH_3$ | $CH_3$ | $CH_3$ | H | $n_D^{22.9}$ = 1.5359 |
| 33 | $CH_3$ | n-$C_3H_7$ | $CH_3$ | n-$C_3H_7$ | $CH_3$ | $CH_3$ | H | $n_D^{22.2}$ = 1.4995 |
| 34 | $C_2H_5$ | n-$C_3H_7$ | $CH_3$ | n-$C_3H_7$ | $CH_3$ | $CH_3$ | H | $n_D^{22.1}$ = 1.4951 |
| 35 | $CH_2$=CH-$CH_2$- | n-$C_3H_7$ | $CH_3$ | n-$C_3H_7$ | $CH_3$ | $CH_3$ | H | $n_D^{22.7}$ = 1.4975 |

Tabelle 2: (Fortsetzung)

| Beispiel Nr. | $R^1$ | $R^2$ | $R^3$ | $R^4$ | $R^5$ | $R^6$ | $R^7$ | physikal. Daten |
|---|---|---|---|---|---|---|---|---|
| 36 | 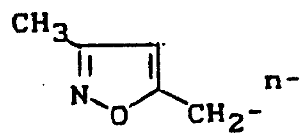 | $n-C_3H_7$ | $CH_3$ | $n-C_3H_7$ | $CH_3$ | $CH_3$ | H | $n_D^{22.2}$ = 1.5073 |
| 37 | 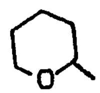 | $n-C_3H_7$ | $CH_3$ | $n-C_3H_7$ | $CH_3$ | $CH_3$ | H | $n_D^{22.1}$ = 1.5014 |
| 38 | $CH_2=CHCH_2-$ | $n-C_3H_7$ | $CH_3$ | $n-C_3H_7$ | $C_6H_5$ | H | H | $n_D^{22}$ = 1.5450 |
| 39 | $C_2H_5$ | $n-C_3H_7$ | $CH_3$ | $n-C_3H_7$ | $C_6H_5$ | H | H | $n_D^{22}$ = 1.5449 |

Tabelle 2: (Fortsetzung)

| Beispiel Nr. | $R^1$ | $R^2$ | $R^3$ | $R^4$ | $R^5$ | $R^6$ | $R^7$ | physikal. Daten |
|---|---|---|---|---|---|---|---|---|
| 40 | $CH_3$ | $n-C_3H_7$ | $CH_3$ | $n-C_3H_7$ | $C_6H_5$ | H | H | $n_D^{22}$ = 1.5516 |
| 41 | $CH_3$ | $n-C_3H_7$ | $CH_3$ | $CH_3$ | $CH_3$- 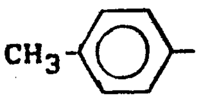 | H | H | Öl |
| 42 | $C_2H_5$ | $n-C_3H_7$ | $CH_3$ | $CH_3$ | $CH_3$- 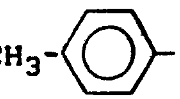 | H | H | $n_D^{20}$ = 1.5431 |
| 43 | $CH_2=CHCH_2-$ | $n-C_3H_7$ | $CH_3$ | $CH_3$ | $CH_3$- 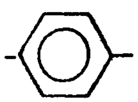 | H | H | $n_D^{22}$ = 1.5462 |
| 44 | $CH_3$ | $n-C_3H_7$ | $CH_3$ | $CH_3$ | $(CH_3)_3C-$ | H | H | $n_D^{22}$ = 1.5118 |

Tabelle 2: (Fortsetzung)

| Bei-spiel Nr. | $R^1$ | $R^2$ | $R^3$ | $R^4$ | $R^5$ | $R^6$ | $R^7$ | physikal. Daten |
|---|---|---|---|---|---|---|---|---|
| 45 | $C_2H_5$ | $n-C_3H_7$ | $CH_3$ | $CH_3$ | $(CH_3)_3C-$ | H | H | $n_D^{22}$ = 1.5019 |
| 46 | $CH_2=CHCH_2$ | $n-C_3H_7$ | $CH_3$ | $CH_3$ | $(CH_3)_3C-$ | H | H | $n_D^{22}$ = 1.5103 |
| 47 | $CH_3$ | $n-C_3H_7$ | $CH_3$ | $CH_3$ | 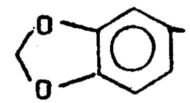 | H | H | Öl |
| 48 | $C_2H_5$ | $n-C_3H_7$ | $CH_3$ | $CH_3$ | 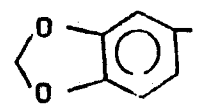 | H | H | Öl |
| 49 | $CH_2=CHCH_2-$ | $n-C_3H_7$ | $CH_3$ | $CH_3$ | 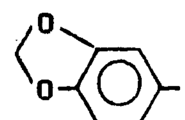 | H | H | Öl |
| 50 | $CH_3$ | $n-C_3H_7$ | $CH_3$ | $CH_3$ | $CH_3O-$ 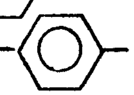 | H | H | $n_D^{22}$ = 1.5509 |

<u>Tabelle 2:</u> (Fortsetzung)

| Bei-spiel Nr. | $R^1$ | $R^2$ | $R^3$ | $R^4$ | $R^5$ | $R^6$ | $R^7$ | physikal. Daten |
|---|---|---|---|---|---|---|---|---|
| 51 | $C_2H_5$ | $n-C_3H_7$ | $CH_3$ | $CH_3$ | 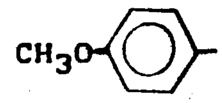 | H | H | $n_D^{22}$ = 1.5436 |
| 52 | $CH_2=CHCH_2-$ | $n-C_3H_7$ | $CH_3$ | $CH_3$ | 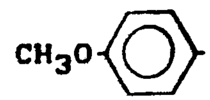 | H | H | $n_D^{22}$ = 1.5539 |
| 53 | $CH_3$ | $n-C_3H_7$ | $CH_3$ | $CH_3$ | 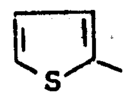 | H | H | $n_D^{20}$ = 1.5528 |
| 54 | $C_2H_5$ | $n-C_3H_7$ | $CH_3$ | $CH_3$ | 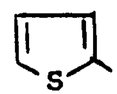 | H | H | $n_D^{20}$ = 1.5518 |
| 55 | $CH_2=CHCH_2-$ | $n-C_3H_7$ | $CH_3$ | $CH_3$ | 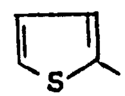 | H | H | $n_D^{20}$ = 1.5554 |

Tabelle 2: (Fortsetzung)

| Beispiel Nr. | $R^1$ | $R^2$ | $R^3$ | $R^4$ | $R^5$ | $R^6$ | $R^7$ | physikal. Daten |
|---|---|---|---|---|---|---|---|---|
| 56 | $CH_2=CHCH_2-$ | $n-C_3H_7$ | $CH_3$ | $CH_3$ | $CH_3$ | $CH_3$ | H | Fp: 81 °C (Zers.) (Natriumsalz) |
| 57 | $CH_2=CHCH_2-$ | $n-C_3H_7$ | $CH_3$ | $CH_3$ | $C_6H_5CH_2O$- 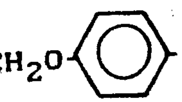 | H | H | Öl |
| 58 | $C_2H_5$ | $n-C_3H_7$ | $CH_3$ | $CH_3$ | $C_6H_5CH_2O$- 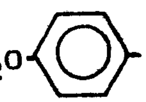 | H | H | Öl |
| 59 | $CH_3$ | $C_2H_5$ | $CH_3$ | $CH_3$ | $CH_3$ | $CH_3$ | H | $n_D^{21}$ = 1.4877 |

Tabelle 2: (Fortsetzung)

| Beispiel Nr. | $R^1$ | $R^2$ | $R^3$ | $R^4$ | $R^5$ | $R^6$ | $R^7$ | physikal. Daten |
|---|---|---|---|---|---|---|---|---|
| 60 | $C_2H_5$ | $C_2H_5$ | $CH_3$ | $CH_3$ | $CH_3$ | $CH_3$ | H | $n_D^{21}$ = 1.5169 |
| 61 | $CH_2=CHCH_2-$ | $C_2H_5$ | $CH_3$ | $CH_3$ | $CH_3$ | $CH_3$ | H | $n_D^{21}$ = 1.5229 |
| 62 | $CH_3OCOCH_2-$ | $n-C_3H_7$ | $CH_3$ | $CH_3$ | $CH_3$ | $CH_3$ | H | $n_D^{21}$ = 1.4991 |
| 63 | $C_2H_5$ | $n-C_3H_7$ | $CH_3$ | $CH_3$ | 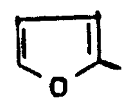 | H | H | Öl |
| 64 | $C_2H_5$ | $n-C_3H_7$ | $CH_3$ | $CH_3OCH_2-$ | $CH_3$ | $CH_3$ | H | $n_D^{22}$ = 1.5050 |
| 65 | $CH_2=CHCH_2-$ | $n-C_3H_7$ | $CH_3$ | $CH_3OCH_2-$ | $CH_3$ | $CH_3$ | H | $n_D^{22}$ = 1.5109 |

Tabelle 2: (Fortsetzung)

| Beispiel Nr. | $R^1$ | $R^2$ | $R^3$ | $R^4$ | $R^5$ | $R^6$ | $R^7$ | physikal. Daten |
|---|---|---|---|---|---|---|---|---|
| 66 | $C_6H_5CH_2$- | n-$C_3H_7$ | $CH_3$ | $CH_3OCH_2$- | $CH_3$ | $CH_3$ | H | $n_D^{22}$ = 1.5284 |
| 67 | $CH_2$=$CHCH_2$- | n-$C_3H_7$ | $C_2H_5$ | $CH_3$ | $CH_3$ | $CH_3$ | H | $n_D^{22}$ = 1.5109 |
| 68 | $C_2H_5$ | n-$C_3H_7$ | $C_2H_5$ | $CH_3$ | $CH_3$ | $CH_3$ | H | $n_D^{22}$ = 1.5061 |
| 69 | $CH_3$ | n-$C_3H_7$ | -$CH_2$- 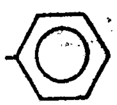 | $CH_3$ | $CH_3$ | $CH_3$ | H | $n_D^{22.8}$ = 1.5332 |
| 70 | $C_2H_5$ | n-$C_3H_7$ | -$CH_2$- 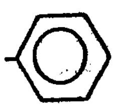 | $CH_3$ | $CH_3$ | $CH_3$ | H | $n_D^{22.8}$ = 1.5327 |

**Tabelle 2:** (Fortsetzung)

| Beispiel Nr. | $R^1$ | $R^2$ | $R^3$ | $R^4$ | $R^5$ | $R^6$ | $R^7$ | physikal. Daten |
|---|---|---|---|---|---|---|---|---|
| 71 | $CH_2=CHCH_2-$ | $n-C_3H_7$ | $-CH_2-$ 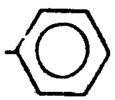 | $CH_3$ | $CH_3$ | $CH_3$ | H | $n_D^{22.8}$ = 1.5362 |
| 72 | $CH_3$ | $n-C_3H_7$ | $-CH_2-$ 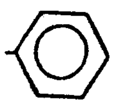 | $CH_3$ | 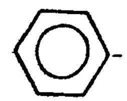 | H | H | $n_D^{22}$ = 1.5729 |
| 73 | $C_2H_5$ | $n-C_3H_7$ | $-CH_2-$ 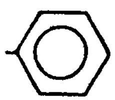 | $CH_3$ | 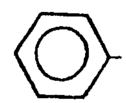 | H | H | $n_D^{22}$ = 1.5702 |
| 74 | $CH_2=CHCH_2-$ | $n-C_3H_7$ | $-CH_2-$ 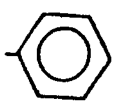 | $CH_3$ | 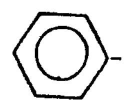 | H | H | $n_D^{22.8}$ = 1.5362 |

Tabelle 2: (Fortsetzung)

| Beispiel Nr. | $R^1$ | $R^2$ | $R^3$ | $R^4$ | $R^5$ | $R^6$ | $R^7$ | physikal. Daten |
|---|---|---|---|---|---|---|---|---|
| 75 | $-CH_2-$ 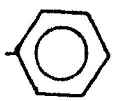 | $n-C_3H_7$ | $-CH_2-$ 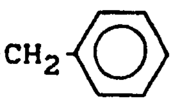 | $CH_3$ | 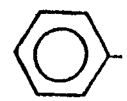 | H | H | $n_D^{22.8}$ = 1.5571 |
| 76 | $-CH_2-$ 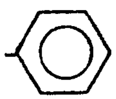 | $n-C_3H_7$ | $CH_3$ | $CH_3OCH_2-$ | 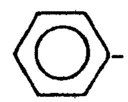 | H | H | $n_D^{23.2}$ = 1.5573 |
| 77 | $CH_3$ | $n-C_3H_7$ | $CH_3$ | $CH_3OCH_2-$ | 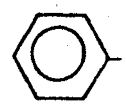 | H | H | $n_D^{23}$ = 1.5384 |
| 78 | $CH_3CH_2-$ | $n-C_3H_7$ | $CH_3$ | $CH_3OCH_2-$ | 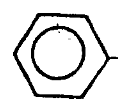 | H | H | $n_D^{23}$ = 1.5365 |

Tabelle 2: (Fortsetzung)

| Bei-spiel Nr. | $R^1$ | $R^2$ | $R^3$ | $R^4$ | $R^5$ | $R^6$ | $R^7$ | physikal. Daten |
|---|---|---|---|---|---|---|---|---|
| 79 | $CH_2=CHCH_2-$ | $n-C_3H_7$ | $CH_3$ | $CH_3OCH_2-$ | 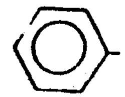 | H | H | $n_D^{23}$ = 1.5380 |
| 80 | $CH_3$ | $n-C_3H_7$ | $CH_3$ | $i-C_3H_7$ | $CH_3$ | $CH_3$ | H | $n_D^{24.5}$ = 1.5023 |
| 81 | $C_2H_5$ | $n-C_3H_7$ | $CH_3$ | $i-C_3H_7$ | $CH_3$ | $CH_3$ | H | $n_D^{24.5}$ = 1.4961 |
| 82 | $CH_2=CH-CH_2-$ | $n-C_3H_7$ | $CH_3$ | $i-C_3H_7$ | $CH_3$ | $CH_3$ | H | $n_D^{24.5}$ = 1.5025 |
| 83 | $C_6H_5-CH_2-$ | $n-C_3H_7$ | $CH_3$ | $i-C_3H_7$ | $CH_3$ | $CH_3$ | H | $n_D^{24.5}$ = 1.5270 |

Tabelle 2: (Fortsetzung)

| Beispiel Nr. | $R^1$ | $R^2$ | $R^3$ | $R^4$ | $R^5$ | $R^6$ | $R^7$ | physikal. Daten |
|---|---|---|---|---|---|---|---|---|
| 84 | $CH_2=CH-CH_2-$ | $n-C_3H_7$ | $CH_3$ | $C_2H_5-S-CH_2-$ | $CH_3$ | $CH_3$ | H | $n_D^{20}$ = 1.5240 |
| 85 | $CH_3$ | $n-C_3H_7$ | $CH_3$ | $C_2H_5$ | $CH_3$ | $CH_3$ | H | $n_D^{24.5}$ = 1.5091 |
| 86 | $C_2H_5$ | $n-C_3H_7$ | $CH_3$ | $C_2H_5$ | $CH_3$ | $CH_3$ | H | $n_D^{24.5}$ = 1.5071 |
| 87 | $CH_2=CH-CH_2-$ | $n-C_3H_7$ | $CH_3$ | $C_2H_5$ | $CH_3$ | $CH_3$ | H | $n_D^{24.5}$ = 1.5137 |
| 88 | $CH_2=CH-CH_2-$ | $C_2H_5$ | $CH_3$ | H | $CH_3$ | $CH_3$ | H | $n_D^{20}$ = 1.5269 |

Tabelle 2: (Fortsetzung)

| Bei-spiel Nr. | $R^1$ | $R^2$ | $R^3$ | $R^4$ | $R^5$ | $R^6$ | $R^7$ | physikal. Daten |
|---|---|---|---|---|---|---|---|---|
| 89 | $CH_2=CH-CH_2-$ | $C_2H_5$ | $CH_3$ | $n-C_3H_7$ | $CH_3$ | $CH_3$ | H | $n_D^{20}$ = 1.5122 |
| 90 | $C_2H_5$ | $C_2H_5$ | $CH_3$ | $n-C_3H_7$ | $CH_3$ | $CH_3$ | H | $n_D^{20}$ = 1.5089 |
| 91 | $CH_3$ | $C_2H_5$ | $CH_3$ | $n-C_3H_7$ | $CH_3$ | $CH_3$ | H | $n_D^{20}$ 1.5103 |
| 92 | $CH_2=CH-CH_2-$ | $CH_3$ | $CH_3$ | $n-C_3H_7$ | $CH_3$ | $CH_3$ | H | $n_D^{20}$ = 1.5106 |
| 93 | $CH_2=CH-CH_2-$ | $C_2H_5$ | $CH_3$ | $CH_3$ | H | H | H | $n_D^{20}$ = 1.5243 |

Tabelle 2: (Fortsetzung)

| Bei-spiel Nr. | $R^1$ | $R^2$ | $R^3$ | $R^4$ | $R^5$ | $R^6$ | $R^7$ | physikal. Daten |
|---|---|---|---|---|---|---|---|---|
| 94 | $CH_2=CH-CH_2-$ | $n-C_{11}H_{23}$ | $CH_3$ | $CH_3$ | $CH_3$ | $CH_3$ | H | $n_D^{20}$ = 1.5009 |
| 95 | $CH_2=CH-CH_2-$ | $CH_3(CH_2)_2-$ | $CH_3$ | $CH_3$ | H | H | H | $n_D^{23.8}$ = 1.5151 |
| 96 | $C_2H_5$ | $CH_3(CH_2)_2-$ | $CH_3$ | $CH_3$ | H | H | H | $n_D^{23.8}$ = 1.5059 |
| 97 | $CH_2=CH-CH_2-$ | $CH_3(CH_2)_2-$ | $CH_3$ | $CH_3(CH_2)_2-$ | H | H | H | $n_D^{20.2}$ = 1.5051 |
| | | $CH_3(CH_2)_2-$ | $CH_3$ | $CH_3(CH_2)_2-$ | H | H | H | $n_D^{20.2}$ = 1.5193 |

Tabelle 2: (Fortsetzung)

| Beispiel Nr. | $R^1$ | $R^2$ | $R^3$ | $R^4$ | $R^5$ | $R^6$ | $R^7$ | physikal. Daten |
|---|---|---|---|---|---|---|---|---|
| 99 | $CH_3$ | $CH_3(CH_2)_2-$ | $CH_3$ | $CH_3(CH_2)_2-$ | H | H | H | $n_D^{20.2}$ = 1.5201 |
| 100 | $CH_2=CH-CH_2-$ | $C_2H_5$ | $CH_3$ | $CH_3OCH_2-$ | $CH_3$ | $CH_3$ | H | $n_D^{20.2}$ = 1.5132 |
| 101 | $C_2H_5$ | $C_2H_5$ | $CH_3$ | $CH_3OCH_2-$ | $CH_3$ | $CH_3$ | H | $n_D^{20.2}$ = 1.5089 |
| 102 | $CH_2=CH-CH_2-$ | $CH_3$ | $CH_3$ | $CH_3OCH_2-$ | $CH_3$ | $CH_3$ | H | $n_D^{20.2}$ = 1.5201 |
| 103 | $CH_2=CH-CH_2-$ | $C_2H_5$ | $CH_3$ | 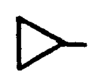 | $CH_3$ | $CH_3$ | H | $n_D^{20.2}$ = 1.5331 |

Tabelle 2: (Fortsetzung)

| Beispiel Nr. | $R^1$ | $R^2$ | $R^3$ | $R^4$ | $R^5$ | $R^6$ | $R^7$ | physikal. Daten |
|---|---|---|---|---|---|---|---|---|
| 104 | $CH_2=CH-CH_2-$ | $CH_3(CH_2)_7-$ | $CH_3$ | 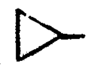 | $CH_3$ | $CH_3$ | H | $n_D^{20.2}$ = 1.5278 |
| 105 | $CH_2=CH-CH_2-$ | $C_2H_5$ | $CH_3$ | $(CH_3)_2CHCH_2-$ | $CH_3$ | $CH_3$ | H | $n_D^{20.2}$ = 1.5092 |
| 106 | $CH_2=CH-CH_2-$ | $CH_3(CH_2)_2-$ | $CH_3$ | $CH_3$ | $CH_3$ | $CH_3$ | H | Fp. 120°C (Zers.) |
| 107 | $CH_2=CH-CH_2-$ | $CH_3(CH_2)_2-$ | $CH_3$ | $(CH_3)_2CHCH_2-$ | $CH_3$ | $CH_3$ | H | $n_D^{20.2}$ = 1.4954 |
| 108 | $C_2H_5$ | $CH_3(CH_2)_2-$ | $CH_3$ | $(CH_3)_2CHCH_2-$ | $CH_3$ | $CH_3$ | H | $n_D^{20.2}$ = 1.5024 |

Tabelle 2: (Fortsetzung)

| Beispiel Nr. | $R^1$ | $R^2$ | $R^3$ | $R^4$ | $R^5$ | $R^6$ | $R^7$ | physikal. Daten |
|---|---|---|---|---|---|---|---|---|
| 109 | $CH_2=CH-CH_2-$ | $CH_3(CH_2)_2-$ | $CH_3$ | 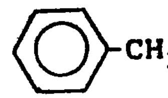 $-CH_2-$ | $CH_3$ | $CH_3$ | H | $n_D^{20.2}$ = 1.5405 |
| 110 | $C_2H_5$ | $CH_3(CH_2)_2-$ | $CH_3$ | 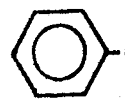 $-CH_2-$ | $CH_3$ | $CH_3$ | H | $n_D^{20.2}$ = 1.5380 |
| 111 | H | $CH_3(CH_2)_2-$ | $CH_3$ | $CH_3$ | $CH_3$ | $CH_3$ | H | Fp. 110°C |
| 112 | $CH_3$ | $CH_3(CH_2)_2-$ | 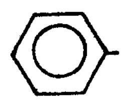 $-CH_2-$ | $CH_3$ | H | H | H | $n_D^{22}$ = 1.5594 |
| 113 | $C_2H_5$ | $CH_3(CH_2)_2-$ | 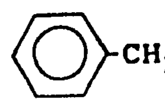 $-CH_2-$ | $CH_3$ | H | H | H | $n_D^{22}$ = 1.5562 |

0172551

Tabelle 2: (Fortsetzung)

| Beispiel Nr. | $R^1$ | $R^2$ | $R^3$ | $R^4$ | $R^5$ | $R^6$ | $R^7$ | physikal. Daten |
|---|---|---|---|---|---|---|---|---|
| 114 | $CH_2=CH-CH_2-$ | $CH_3(CH_2)_2-$ | 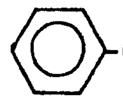 $-CH_2-$ | $CH_3$ | H | H | H | $n_D^{22}$ = 1.5578 |
| 115 | $CH_2=CH-CH_2-$ | $CH_3(CH_2)_2-$ | $CH_3$ | $C_2H_5$ | H | H | H | $n_D^{22.7}$ = 1.5229 |
| 116 | $C_2H_5$ | $CH_3(CH_2)_2-$ | $CH_3$ | $C_2H_5$ | H | H | H | $n_D^{21.9}$ 1.5191 |
| 117 | $CH_3O-N=CH-CH(CH_3)-$ | $CH_3(CH_2)_2-$ | $CH_3$ | $CH_3$ | $CH_3$ | $CH_3$ | H | $n_D^{22.1}$ = 1.5131 |
| 118 | Cl 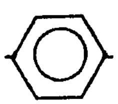 $CH_2$ | $CH_3(CH_2)_2-$ | $CH_3$ | $CH_3$ | $CH_3$ | $CH_3$ | H | Öl |

Tabelle 2: (Fortsetzung)

| Beispiel Nr. | $R^1$ | $R^2$ | $R^3$ | $R^4$ | $R^5$ | $R^6$ | $R^7$ | physikal. Daten |
|---|---|---|---|---|---|---|---|---|
| 119 | 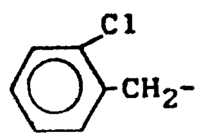 | $CH_3(CH_2)_2$- | $CH_3$ | $CH_3$ | $CH_3$ | $CH_3$ | H | $n_D^{22.8}$ = 1.5409 |
| 120 | 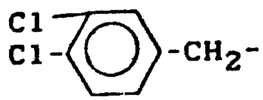 | $CH_3(CH_2)_2$- | $CH_3$ | $CH_3$ | $CH_3$ | $CH_3$ | H | $n_D^{22.8}$ = 1.5502 |
| 121 | 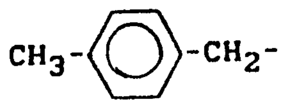 | $CH_3(CH_2)_2$- | $CH_3$ | $CH_3$ | $CH_3$ | $CH_3$ | H | $n_D^{22.8}$ = 1.5366 |
| 122 | 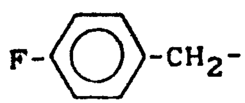 | $CH_3(CH_2)_2$- | $CH_3$ | $CH_3$ | $CH_3$ | $CH_3$ | H | Fp. 54-55°C |

Le A 23 265

Tabelle 2: (Fortsetzung)

| Beispiel Nr. | $R^1$ | $R^2$ | $R^3$ | $R^4$ | $R^5$ | $R^6$ | $R^7$ | physikal. Daten |
|---|---|---|---|---|---|---|---|---|
| 123 | NC- 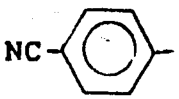 -$CH_2$ | $CH_3(CH_2)_2$- | $CH_3$ | $CH_3$ | $CH_3$ | $CH_3$ | H | Fp. 90°-91°C |
| 124 | $C_2H_5$ | $CH_3(CH_2)_2$- | $(CH_3)_2CH$- | $CH_3$ | $CH_3$ | $CH_3$ | H | $n_D^{23}$ = 1.5031 |
| 125 | $CH_2$=CH-$CH_2$- | $CH_3(CH_2)_2$- | $(CH_3)_2CH$- | $CH_3$ | $CH_3$ | $CH_3$ | H | $n_D^{23}$ = 1.5079 |
| 126 | $(CH_3)_2CH$- | $CH_3(CH_2)_2$- | $(CH_3)_2CH$- | $CH_3$ | $CH_3$ | $CH_3$ | H | $n_D^{23}$ = 1.4993 |
| 127 | 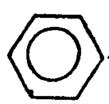 -$CH_2$- | $CH_3(CH_2)_2$- | $(CH_3)_2CH$- | $CH_3$ | $CH_3$ | $CH_3$ | H | $n_D^{23}$ = 1.5324 |

Tabelle 2: (Fortsetzung)

| Bei-spiel Nr. | $R^1$ | $R^2$ | $R^3$ | $R^4$ | $R^5$ | $R^6$ | $R^7$ | physikal. Daten |
|---|---|---|---|---|---|---|---|---|
| 128 | $CH_2$=CH-$CH_2$ | $CH_3(CH_2)_2$- | $CH_2$=CH-$CH_2$- | $CH_3$ | $CH_3$ | $CH_3$ | H | $n_D^{22.7}$ = 1.5191 |
| 129 | Cl-CH=CH-$CH_2$- | $CH_3(CH_2)_2$- | $CH_3$ | $CH_3$ | $CH_3$ | $CH_3$ | H | $n_D^{23}$ = 1.5245 |
| 130 | $O_2N$-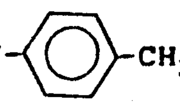-$CH_2$- | $CH_3(CH_2)_2$- | $CH_3$ | $CH_3$ | $CH_3$ | $CH_3$ | H | amorph |
| 131 | $CH_2$=CH-$CH_2$- | 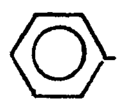 | $CH_3$ | $CH_3$ | $CH_3$ | $CH_3$ | H | Fp. 159°C |
| 132 | $CH_3$ | $CH_3(CH_2)_2$- | $CH_3$ | $CH_3$ | $CH_3S$-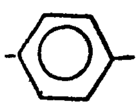 | H | H | $n_D^{23}$ = 1.5627 |
| 133 | $C_2H_5$ | $CH_3(CH_2)_2$- | $CH_3$ | $CH_3$ | $CH_3S$-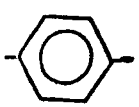 | H | H | $n_D^{23}$ = 1.5741 |

0172551

Tabelle 2: (Fortsetzung)

| Beispiel Nr. | $R^1$ | $R^2$ | $R^3$ | $R^4$ | $R^5$ | $R^6$ | $R^7$ | physikal. Daten |
|---|---|---|---|---|---|---|---|---|
| 134 | $CH_2=CH-CH_2-$ | $CH_3(CH_2)_2-$ | $CH_3$ | $CH_3$ | $CH_3S-$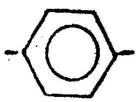 | H | H | $n_D^{23}$ = 1.5769 |
| 135 | $(CH_3)_2CH-$ | $CH_3(CH_2)_2-$ | $CH_3$ | $CH_3$ | $CH_3S-$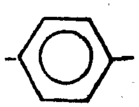 | H | H | $n_D^{23}$ = 1.5639 |
| 136 | 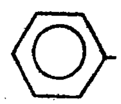$-CH_2-$ | $CH_3(CH_2)_2-$ | $CH_3$ | $CH_3$ | $CH_3S-$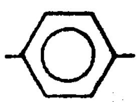 | H | H | $n_D^{23}$ = 1.5928 |
| 137 | $C_2H_5$ | $CH_3(CH_2)_2-$ | $CH_3$ | $CH_3$ | 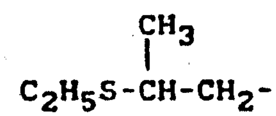 | H | H | $n_D^{22}$ = 1.5170 |

Tabelle 2: (Fortsetzung)

| Bei-spiel Nr. | $R^1$ | $R^2$ | $R^3$ | $R^4$ | $R^5$ | $R^6$ | $R^7$ | physikal. Daten |
|---|---|---|---|---|---|---|---|---|
| 138 | $CH_2=CH-CH_2-$ | $CH_3(CH_2)_2-$ | $CH_3$ | $CH_3$ | $C_2H_5-S-CH(CH_3)-CH_2-$ | H | H | $n_D^{23.4}$ = 1.5302 |
| 139 | $C_2H_5$ | $CH_3(CH_2)_2-$ | $CH_3$ | $CH_3$ | H | $-(CH_2)_4-$ | | $n_D^{23}$ = 1.5289 |
| 140 | $CH_2=CH-CH_2-$ | $CH_3(CH_2)_2-$ | $CH_3$ | $CH_3$ | H | $-(CH_2)_4-$ | | $n_D^{23}$ = 1.5309 |
| 141 | 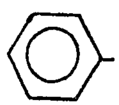 $-CH_2-$ | $CH_3(CH_2)_2-$ | $CH_3$ | $CH_3$ | H | $-(CH_2)_4-$ | | $n_D^{23}$ = 1.5551 |

Tabelle 2: (Fortsetzung)

| Bei-spiel Nr. | $R^1$ | $R^2$ | $R^3$ | $R^4$ | $R^5$ | $R^6$ | $R^7$ | physikal. Daten |
|---|---|---|---|---|---|---|---|---|
| 142 | $(CH_3)_2CH-$ | $CH_3(CH_2)_2-$ | $CH_3$ | $CH_3$ | H | $-(CH_2)_4-$ | | $n_D^{23}$ = 1.5236 |
| 143 | 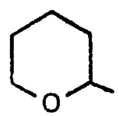 | $CH_3(CH_2)_2-$ | $CH_3$ | $CH_3$ | H | $-(CH_2)_4-$ | | $n_D^{23}$ = 1.5273 |
| 144 | $C_2H_5$ | 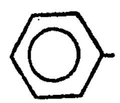 | $CH_3$ | $CH_3$ | $CH_3$ | $CH_3$ | H | Fp. 156°-157°C |
| 145 | $CH_3$ | $CH_3(CH_2)_2-$ | $CH_3$ | $CH_3$ | 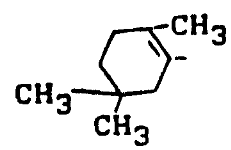 | H | H | $n_D^{23}$ = 1.5236 |

Tabelle 2: (Fortsetzung)

| Bei-spiel Nr. | $R^1$ | $R^2$ | $R^3$ | $R^4$ | $R^5$ | $R^6$ | $R^7$ | physikal. Daten |
|---|---|---|---|---|---|---|---|---|
| 146 | $C_2H_5$ | $CH_3(CH_2)_2-$ | $CH_3$ | $CH_3$ | 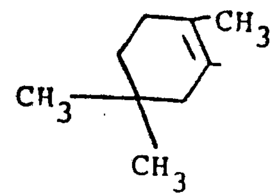 | H | H | $n_D^{23}$ = 1.5281 |
| 147 | $(CH_3)_2CH-$ | $CH_3(CH_2)_2-$ | $CH_3$ | $CH_3$ | 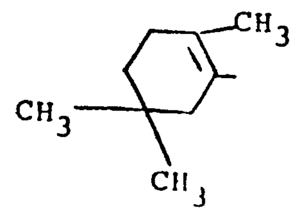 | H | H | $n_D^{23}$ = 1.5233 |
| 148 | 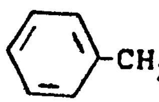$-CH_2-$ | $CH_3(CH_2)_2-$ | $CH_3$ | $CH_3$ | 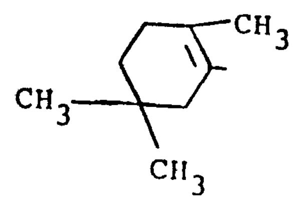 | H | H | $n_D^{23}$ = 1.5470 |

Tabelle 2: (Fortsetzung)

| Bei-spiel Nr. | $R^1$ | $R^2$ | $R^3$ | $R^4$ | $R^5$ | $R^6$ | $R^7$ | physikal. Daten |
|---|---|---|---|---|---|---|---|---|
| 149 | $C_2H_5$ | Cl-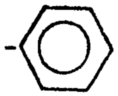- | $CH_3$ | $CH_3$ | $CH_3$ | $CH_3$ | H | Fp. 174-177°C |
| 150 | $CH_2$=CH-$CH_2$- | Cl-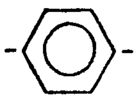- | $CH_3$ | $CH_3$ | $CH_3$ | $CH_3$ | H | Fp. 157-160°C |
| 151 | 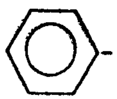-$CH_2$- | Cl-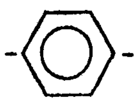- | $CH_3$ | $CH_3$ | $CH_3$ | $CH_3$ | H | Fp. 75-85°C |
| 152 | $C_2H_5$ | $CH_3$-$(CH_2)_2$- | $CH_3$ | $CH_3$ | 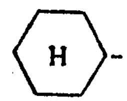- | H | H | $n_D^{23}$ = 1.5217 |
| 153 | $CH_2$=CH-$CH_2$- | $CH_3(CH_2)_2$- | $CH_3$ | $CH_3$ | 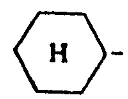- | H | H | $n_D^{23}$ = 1.5245 |

Tabelle 2: (Fortsetzung)

| Beispiel Nr. | $R^1$ | $R^2$ | $R^3$ | $R^4$ | $R^5$ | $R^6$ | $R^7$ | physikal. Daten |
|---|---|---|---|---|---|---|---|---|
| 154 | 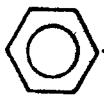 $-CH_2-$ | $CH_3(CH_2)_2-$ | $CH_3$ | $CH_3$ | 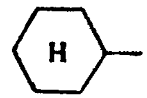 | H | H | $n_D^{23}$ = 1.5452 |
| 155 | $CH_3$ | 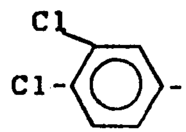 | $CH_3$ | $CH_3$ | $CH_3$ | $CH_3$ | H | Fp. 127–139°C |
| 156 | $C_2H_5$ | 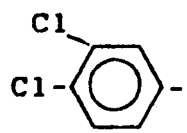 | $CH_3$ | $CH_3$ | $CH_3$ | $CH_3$ | H | Fp. 123-128°C |
| 157 | $(CH_3)_2CH-$ | 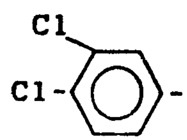 | $CH_3$ | $CH_3$ | $CH_3$ | $CH_3$ | H | Fp. 110-113°C* 117-120°C* |
| 158 | $CH_2=CH-CH_2-$ | 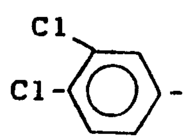 | $CH_3$ | $CH_3$ | $CH_3$ | $CH_3$ | H | Fp. 125-131°C |

Tabelle 2: (Fortsetzung)

| Bei-spiel Nr. | $R^1$ | $R^2$ | $R^3$ | $R^4$ | $R^5$ | $R^6$ | $R^7$ | physikal. Daten |
|---|---|---|---|---|---|---|---|---|
| 159 | 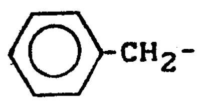 | 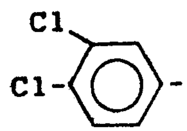 | $CH_3$ | $CH_3$ | $CH_3$ | $CH_3$ | H | Fp. 109-111°C* Fp. 145-148°C* |
| 160 | $Cl-CH=CH-CH_2-$ | $CH_3(CH_2)_2-$ | $CH_3$ | $CH_3$ | $C_2H_5S\overset{CH_3}{\overset{\mid}{C}}HCH_2-$ | H | H | $n_D^{22}$ = 1.5254 |
| 161 | $CH_2=CH-CH_2-$ | $CH_3(CH_2)_2-$ | $CH_3$ | $CF_3$ | $CH_3$ | $CH_3$ | H | $n_D^{22}$ = 1.4874 |

<u>Tabelle 2:</u> (Fortsetzung)

| Beispiel Nr. | $R^1$ | $R^2$ | $R^3$ | $R^4$ | $R^5$ | $R^6$ | $R^7$ | physikal. Daten |
|---|---|---|---|---|---|---|---|---|
| 162 | $Cl-CH=CH-CH_2-$ | $CH_3(CH_2)_2-$ | $CH_3$ | $CF_3$ | $CH_3$ | $CH_3$ | H | $n_D^{22}$ = 1.4978 |
| 163 | $CH_2=CH-CH_2-$ | $CH_3(CH_2)_2-$ | $CH_3$ | $CH_3$ | 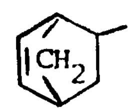 | H | H | $n_D^{22}$ = 1.5319 |
| 164 | $Cl-CH=CH-CH_2-$ | $CH_3(CH_2)_2-$ | $CH_3$ | $CH_3$ | 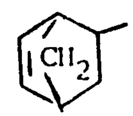 | H | H | Öl |
| 165 | $(C_6H_5)_3C-$ | $CH_3(CH_2)_2-$ | $CH_3$ | $CH_3$ | $CH_3$ | $CH_3$ | H | Öl |
| 166 | $CH_3-\overset{Cl}{\overset{\mid}{C}}=CH-CH_2-$ | $CH_3(CH_2)_2-$ | $CH_3$ | $CH_3$ | $CH_3$ | $CH_3$ | H | $n_D^{22.4}$ 1.5244 |

Tabelle 2: (Fortsetzung)

| Bei-spiel Nr. | $R^1$ | $R^2$ | $R^3$ | $R^4$ | $R^5$ | $R^6$ | $R^7$ | physikal. Daten |
|---|---|---|---|---|---|---|---|---|
| 167 | $CH_2=CH-CH_2-$ | $(CH_3)_2CHCH_2-$ | $CH_3$ | $CH_3$ | $CH_3$ | $CH_3$ | H | $n_D^{22}$ = 1.5128 |
| 168 | $Cl-CH=CH-CH_2-$ | $(CH_3)_2CHCH_2-$ | $CH_3$ | $CH_3$ | $CH_3$ | $CH_3$ | H | $n_D^{22.8}$ 1.5237 |
| 169 | $CH_3-\overset{Cl}{\overset{\vert}{C}}=CH-CH_2-$ | $(CH_3)_2CHCH_2-$ | $CH_3$ | $CH_3$ | $CH_3$ | $CH_3$ | H | $n_D^{22.8}$ = 1.5213 |
| 170 | $CH_3$ | $CH_3(CH_2)_2-$ | $CH_3$ | $CH_3$ | $(CH_3)_2CH-$ | H | H | $n_D^{22.4}$ 1.5186 |

Tabelle 2: (Fortsetzung)

| Beispiel Nr. | $R^1$ | $R^2$ | $R^3$ | $R^4$ | $R^5$ | $R^6$ | $R^7$ | physikal. Daten |
|---|---|---|---|---|---|---|---|---|
| 171 | $C_2H_5$ | $CH_3(CH_2)_2-$ | $CH_3$ | $CH_3$ | $(CH_3)_2CH-$ | H | H | $n_D^{22.4}$ = 1.5139 |
| 172 | $CH_2=CH-CH_2-$ | $CH_3(CH_2)_2-$ | $CH_3$ | $CH_3$ | $(CH_3)_2CH-$ | H | H | $n_D^{22.4}$ = 1.5192 |
| 173 | $Cl-CH=CH-CH_2-$ | $CH_3(CH_2)_2-$ | $CH_3$ | $CH_3$ | $(CH_3)_2CH-$ | H | H | $n_D^{22.4}$ = 1.5241 |
| 174 | $CH_3$ | 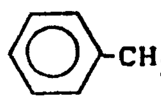$-CH_2-CH_2-$ | $CH_3$ | $CH_3$ | $CH_3$ | $CH_3$ | H | Fp. 71-73°C |
| 175 | $C_2H_5$ | 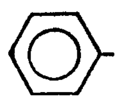$-CH_2-CH_2-$ | $CH_3$ | $CH_3$ | $CH_3$ | $CH_3$ | H | Fp. 56-57°C |
| 176 | $CH_2=CH-CH_2-$ | 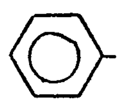$-CH_2-CH_2-$ | $CH_3$ | $CH_3$ | $CH_3$ | $CH_3$ | H | $n_D^{22.4}$ = 1.5501 |

Tabelle 2: (Fortsetzung)

| Beispiel Nr. | $R^1$ | $R^2$ | $R^3$ | $R^4$ | $R^5$ | $R^6$ | $R^7$ | physikal. Daten |
|---|---|---|---|---|---|---|---|---|
| 177 | $(C_6H_5)_3C-$ | $CH_3(CH_2)_2-$ | $CH_3$ | $CH_3$ | $CH_3$ | $CH_3$ | H | $n_D^{22}$ = 1.5540 |
| 178 | $CH_3$ | 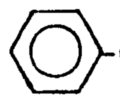$-CH_2-$ | $CH_3$ | $CH_3$ | $CH_3$ | $CH_3$ | H | $n_D^{22}$ = 1.5422 |
| 179 | $C_2H_5$ | 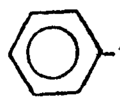$-CH_2-$ | $CH_3$ | $CH_3$ | $CH_3$ | $CH_3$ | H | Fp. 74-76°C |
| 180 | $CH_2=CH-CH_2$ | 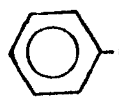$-CH_2-$ | $CH_3$ | $CH_3$ | $CH_3$ | $CH_3$ | H | Öl |
| 181 | $C_2H_5$ | $CH_3(CH_2)_3-$ | $CH_3$ | $CH_3$ | $CH_3$ | $CH_3$ | H | $n_D^{22}$ = 1.5080 |
| 182 | $CH_3$ | $CH_3(CH_2)_3-$ | $CH_3$ | $CH_3$ | $CH_3$ | $CH_3$ | H | $n_D^{22}$ = 1.5115 |

Tabelle 2: (Fortsetzung)

| Beispiel Nr. | $R^1$ | $R^2$ | $R^3$ | $R^4$ | $R^5$ | $R^6$ | $R^7$ | physikal. Daten |
|---|---|---|---|---|---|---|---|---|
| 183 | $CH_2$=CH-$CH_2$ | $CH_3(CH_2)_2$- | $CH_3$ | $CH_3$ | $CH_3$ | $CH_3$ | H | $n_D^{22}$ = 1.5127 |
| 184 | Cl-CH=CH-$CH_2$- | $CH_3(CH_2)_3$- | $CH_3$ | $CH_3$ | $CH_3$ | $CH_3$ | H | $n_D^{22}$ = 1.5251 |
| 185 | $CH_2$=CH-$CH_2$- | $(CH_3)_2$CH- | $CH_3$ | $CH_3$ | $CH_3$ | $CH_3$ | H | $n_D^{22}$ = 1.5162 |
| 186 | $C_2H_5$ | $(CH_3)_2$CH- | $CH_3$ | $CH_3$ | $CH_3$ | $CH_3$ | H | $n_D^{22}$ = 1.5077 |

Durch * sind jeweils verschiedene isomere oder tautomere Formen gekennzeichnet.

Herstellung der Ausgangsverbindungen:

a) Beispiel (II-1)

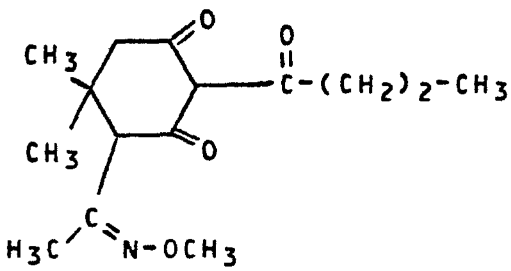

(II-1)

Zu 60 g (0,28 Mol) 5,5-Dimethyl-4-(1-methoximino-ethyl)-cyclohexan-1,3-dion und 45,6 g (0,3 Mol) Diazabicycloundecen (DBU) in 400 ml Toluol tropft man bei 0°C unter Rühren 30,9 g (0,29 Mol) Buttersäurechlorid und rührt nach beendeter Zugabe weitere 2 Stunden bei Raumtemperatur nach. Anschließend wäscht man die organische Phase mit 1%-iger Salzsäure und Wasser, trocknet über Natriumsulfat, setzt ca. 0,1 g 4-(N,N-Dimethylamino)-pyridin zu und erhitzt für 3 Stunden unter Rückfluß. Die erkaltete Reaktionsmischung wird mit verdünnter Salzsäure und Wasser gewaschen, über Magnesiumsulfat getrocknet und im Vakuum eingeengt. Man erhält 69,7 g (87 % der Theorie) an 2-Butyryl-5,5-dimethyl-4-(1-methoximino-ethyl)-cyclohexan-1,3-dion vom Brechungsindex $n_D^{22,4}$= 1,5034.

In entsprechender Weise und gemäß den allgemeinen Herstellungsangaben erhält man die folgenden Verbindungen der Formel (II):

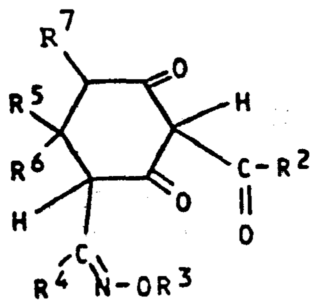

(II)

Als physikalische Daten sind in Tabelle 3 angegeben:

Schmelzpunkt (Fp. in °C)
Brechungsindex ($n_D$) [Temperatur in °C]
chemische Verschiebung $\delta$ in ppm bezogen auf $^1$H-NMR in $CDCl_3$

Tabelle 3:

| Beispiel Nr. | $R^2$ | $R^3$ | $R^4$ | $R^5$ | $R^6$ | $R^7$ | physikal. Daten |
|---|---|---|---|---|---|---|---|
| II-2 | $n-C_3H_7$ | $CH_3$ | $CH_3$ | 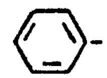 | H | H | 1,5410[22,1] |
| II-3 | $n-C_3H_7$ | $CH_3$ | H | $CH_3$ | $CH_3$ | H | 3,84δ($OCH_3$) |
| II-4 | $n-C_3H_7$ | $CH_3$ | $CH_3$ | 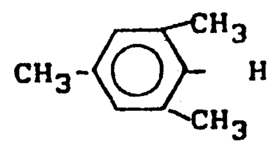 | H | H | 3,69δ($OCH_3$) |
| II-5 | $C_2H_5$ | $CH_3$ | $CH_3$ | 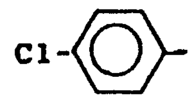 | H | H | 3,71δ($OCH_3$) |
| II-6 | $n-C_3H_7$ | $CH_3$ | $CH_3$ | 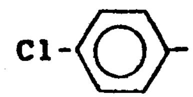 | H | H | 3,71δ($OCH_3$) |
| II-7 | $C_2H_5$ | $CH_3$ | $CH_3$ | 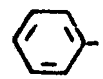 | H | H | 3,69δ($OCH_3$) |
| II-8 | $CH_3$ | $CH_3$ | $CH_3$ | 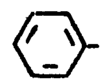 | H | H | 3,69δ($OCH_3$) |
| II-9 | $C_2H_5$ | $CH_3$ | $CH_3$ | $CH_3$ | $CH_3$ | H | |

Tabelle 3: (Fortsetzung)

| Beispiel Nr. | $R^2$ | $R^3$ | $R^4$ | $R^5$ | $R^6$ | $R^7$ | physikal. Daten |
|---|---|---|---|---|---|---|---|
| II-10 | $CH_3$ | $CH_3$ | $CH_3$ | $CH_3$ | $CH_3$ | H | 3,81δ($OCH_3$) |
| II-11 | n-$C_3H_7$ | $CH_3$ | n-$C_3H_7$ | $CH_3$ | $CH_3$ | H | 3,82δ($OCH_3$) |
| II-12 | n-$C_3H_7$ | $CH_3$ | n-$C_3H_7$ | 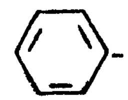 | H | H | 3,78δ($OCH_3$) |
| II-13 | n-$C_3H_7$ | $CH_3$ | $CH_3$ | 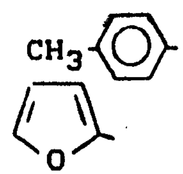 | H | H | 1,5466 [22,9] |
| II-14 | n-$C_3H_7$ | $CH_3$ | $CH_3$ |  | H | H | 1,5298 [22] |
| II-15 | n-$C_3H_7$ | $CH_3$ | $CH_3$ | $(CH_3)_3C$- | H | H | 1,5062 [22] |
| II-16 | n-$C_3H_7$ | $CH_3$ | $CH_3$ | 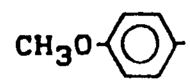 | H | H | 1,5524 [22] |
| II-17 | n-$C_3H_7$ | $CH_3$ | $CH_3$ | 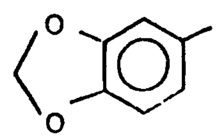 | H | H | 3,74δ ($OCH_3$) |

Tabelle 3: (Fortsetzung)

| Bei-spiel Nr. | $R^2$ | $R^3$ | $R^4$ | $R^5$ | $R^6$ | $R^7$ | physikal. Daten |
|---|---|---|---|---|---|---|---|
| II-18 | $n-C_3H_7$ | $CH_3$ | $CH_3$ | 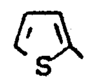 | H | H | 3,75δ$(OCH_3)$ |
| II-19 | $n-C_3H_7$ | $C_2H_5$ | $CH_3$ | $CH_3$ | $CH_3$ | H | 4,04δ$[OCH_2-]$ |
| II-20 | $n-C_3H_7$ | $-CH_2-CH=CH_2$ | $CH_3$ | $CH_3$ | $CH_3$ | H | 1,5087 [22] |
| II-21 | $n-C_3H_7$ | $CH_3$ | $CH_3$ | 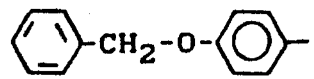 | H | H | 3,69δ$(OCH_3)$ |
| II-22 | $n-C_3H_7$ | $CH_3$ | $CH_3OCH_2-$ | $CH_3$ | $CH_3$ | H | 3,77δ$(OCH_3)$ |
| II-23 | 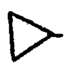 | $CH_3$ | $CH_3$ | $CH_3$ | $CH_3$ | H | 3,81δ$(OCH_3)$ |
| II-24 | $n-C_3H_7$ | $-C_2H_5$ | $CH_3$ | $CH_3$ | $CH_3$ | H | 1,90δ$(CH_3)$ |
| II-25 | $n-C_3H_7$ | $-CH_2-$ 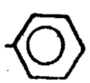 | $CH_3$ | $CH_3$ | $CH_3$ | H | 1,94δ$(CH_3)$ |

Tabelle 3: (Fortsetzung)

| Beispiel Nr. | $R^2$ | $R^3$ | $R^4$ | $R^5$ | $R^6$ | $R^7$ | physikal. Daten |
|---|---|---|---|---|---|---|---|
| II-26 | n-$C_3H_7$ | $-CH_2-$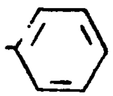 | $CH_3$ | 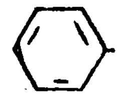 | H | H | 4,95δ($OCH_2$-) |
| II-27 | n-$C_3H_7$ | $CH_3$ | $CH_3OCH_2$- | 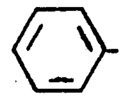 | H | H | 7,19δ(Phenyl) |
| II-28 | n-$C_3H_7$ | $CH_3$ | $(CH_3)_2CH$- | $CH_3$ | $CH_3$ | H | 3,73δ($OCH_3$) |
| II-29 | n-$C_3H_7$ | $CH_3$ | $C_2H_5$-S-$CH_2$- | $CH_3$ | $CH_3$ | H | 3,76δ($OCH_3$) |
| II-30 | n-$C_3H_7$ | $CH_3$ | $CH_3CH_2$- | $CH_3$ | $CH_3$ | H | 3,74δ($OCH_3$) |
| II-31 | n-$C_3H_7$ | $CH_3$ | $CH_3$ | H | H | H | 3,82δ($OCH_3$) |
| II-32 | -$C_2H_5$ | $CH_3$ | H | $CH_3$ | $CH_3$ | H | 3,83δ($OCH_3$) |
| II-33 | $CH_3$ | $CH_3$ | n-$C_3H_7$ | $CH_3$ | $CH_3$ | H | 3,72δ($OCH_3$) |
| II-34 | -$C_2H_5$ | $CH_3$ | n-$C_3H_7$ | $CH_3$ | $CH_3$ | H | 3,73δ($OCH_3$) |
| II-35 | -$C_2H_5$ | $CH_3$ | $CH_3$ | H | H | H | 3,83δ($OCH_3$) |
| II-36 | n-$C_{11}H_{23}$ | $CH_3$ | $CH_3$ | $CH_3$ | $CH_3$ | H | 1,4921 (20) |
| II-37 | n-$C_3H_7$ | $CH_3$ | n-$C_3H_7$ | H | H | H | 3,81δ($OCH_3$) |
| II-38 | n-$C_3H_7$ | $CH_3$ | $CH_3OCH_2$- | H | H | H | 3,81δ($OCH_3$) |

Tabelle 3: (Fortsetzung)

| Bei-spiel Nr. | $R^2$ | $R^3$ | $R^4$ | $R^5$ | $R^6$ | $R^7$ | physikal. Daten |
|---|---|---|---|---|---|---|---|
| II-39 | $CH_3$ | $CH_3$ | $CH_3OCH_2-$ | $CH_3$ | $CH_3$ | H | 3,79δ($OCH_3$) |
| II-40 | $-C_2H_5$ | $CH_3$ | $CH_3OCH_2-$ | $CH_3$ | $CH_3$ | H | 3,79δ($OCH_3$) |
| II-41 | $-C_2H_5$ | $CH_3$ | 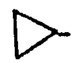 | $CH_3$ | $CH_3$ | H | 3,76δ($OCH_3$) |
| II-42 | $n-C_3H_7$ | $CH_3$ | 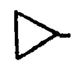 | $CH_3$ | $CH_3$ | H | 3,75δ($OCH_3$) |
| II-43 | $n-C_3H_7$ | $CH_3$ | 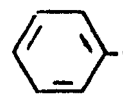$-CH_2-$ | $CH_3$ | $CH_3$ | H | 1,5457[20,2] |
| II-44 | $n-C_3H_7$ | $CH_3$ | $(CH_3)_2CH-CH_2-$ | $CH_3$ | $CH_3$ | H | 1,5003[20,2] |
| II-45 | $-C_2H_5$ | $CH_3$ | $(CH_3)_2CH-CH_2-$ | $CH_3$ | $CH_3$ | H | 3,71δ[$OCH_3$] |
| II-46 | $C_2H_5$ | $CH_3$ | $(CH_3)_2CH-$ | $CH_3$ | $CH_3$ | H | 1,5037 [20,2] |
| II-47 | $n-C_3H_7$ | 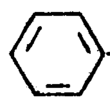$-CH_2-$ | $CH_3$ | H | H | H | 5,04δ($OCH_2-$) |
| II-48 | $n-C_3H_7$ | $CH_3$ | $C_2H_5$ | H | H | H | 3,80δ($OCH_3$) |

Tabelle 3: (Fortsetzung)

| Beispiel Nr. | $R^2$ | $R^3$ | $R^4$ | $R^5$ | $R^6$ | $R^7$ | physikal. Daten |
|---|---|---|---|---|---|---|---|
| II-49 | n-$C_3H_7$ | $-CH_2-CH=CH_2$ | $CH_3$ | $CH_3$ | $CH_3$ | H | 1,94δ($CH_3$) |
| II-50 | n-$C_3H_7$ | $-CH(CH_3)_2$ | $CH_3$ | $CH_3$ | $CH_3$ | H | 1,90δ($CH_3$) |
| II-51 | n-$C_3H_7$ | $CH_3$ | $CH_3$ | 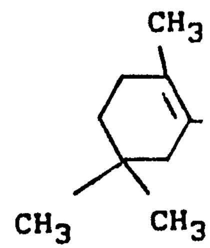 | H | H | 1,5317[23,1] |
| II-52 | n-$C_3H_7$ | $CH_3$ | $CH_3$ | 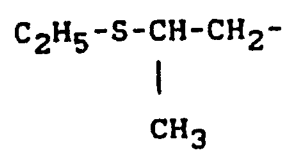 | H | H | 3,86δ($OCH_3$) |
| II-53 | 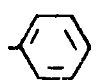 | $CH_3$ | $CH_3$ | $CH_3$ | $CH_3$ | H | Fp. 95°C |
| II-54 | n-$C_3H_7$ | $CH_3$ | $CH_3$ | $CH_3S$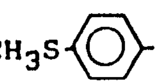 | H | H | 1,5803[23] |

Tabelle 3: (Fortsetzung)

| Beispiel Nr. | $R^2$ | $R^3$ | $R^4$ | $R^5$ | $R^6$ | $R^7$ | physikal. Daten |
|---|---|---|---|---|---|---|---|
| II-55 | 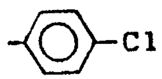 | $CH_3$ | $CH_3$ | $CH_3$ | $CH_3$ | H | Fp. 70-71 |
| II-56 | 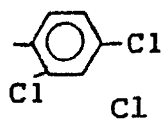 | $CH_3$ | $CH_3$ | $CH_3$ | $CH_3$ | H | 3,83δ($OCH_3$) |
| II-57 | 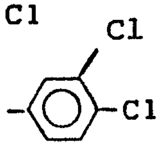 | $CH_3$ | $CH_3$ | $CH_3$ | $CH_3$ | H | 3,78δ($OCH_3$) |
| II-58 | n-$C_3H_7$ | $CH_3$ | $CH_3$ | 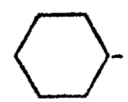 | H | H | 1,5223 [23] |
| II-59 | n-$C_3H_7$ | $CF_3$ | $CH_3$ | $CH_3$ | $CH_3$ | H | 3,96δ($OCH_3$) |
| II-60 | n-$C_3H_7$ | $CH_3$ | $CH_3$ | 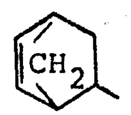 | H | H | 3,81δ($OCH_3$) |
| II-61 | $-CH_2-CH(CH_3)_2$ | $CH_3$ | $CH_3$ | $CH_3$ | $CH_3$ | H | 1,5050[22,8] |
| II-62 | $-CH_2CH_2-$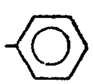 | $CH_3$ | $CH_3$ | $CH_3$ | $CH_3$ | H | 1,5509(22,8) |

Tabelle 3: (Fortsetzung)

| Beispiel Nr. | $R^2$ | $R^3$ | $R^4$ | $R^5$ | $R^6$ | $R^7$ | physikal. Daten |
|---|---|---|---|---|---|---|---|
| II-63 | n-$C_3H_7$ | $CH_3$ | $CH_3$ | $(CH_3)_2CH-$ | H | H | 1,5162 (22,8) |
| II-64 | $-CH(CH_3)_2$ | $CH_3$ | $CH_3$ | $CH_3$ | $CH_3$ | H | 1,5089 (22) |
| II-65 | n-$C_4H_9$ | $CH_3$ | $CH_3$ | $CH_3$ | $CH_3$ | H | 1,5067 (22) |
| II-66 | $-CH_2-$ 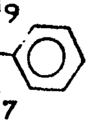 | $CH_3$ | $CH_3$ | $CH_3$ | $CH_3$ | H | Fp. 54-56 |
| II-67 | n-$C_3H_7$ | $CH_3$ | $CH_3$ | H | $-(CH_2)_6-$ | | 1,5311[23] |

b) Beispiel (V-1)

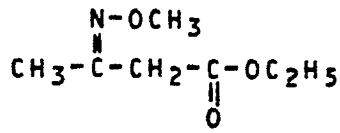

(V-1)

1000 g (7,7 Mol) Acetessigsäureethylester in 1,6 l 66-%-igem wäßrigem Ethanol werden nacheinander mit 854,5 g (10,25 Mol) O-Methylhydroxylamin-Hydrochlorid und 832,7 g (10,25 Mol) Natriumacetat versetzt und anschließend für 5 Stunden unter Rückfluß erhitzt.

Die erkaltete Mischung wird im Vakuum eingeengt, der Rückstand in Wasser aufgenommen und mehrfach mit Dichlormethan extrahiert; die vereinigten organischen Phasen werden über Magnesiumsulfat getrocknet und nach Einengen im Wasserstrahlvakuum destilliert. Man erhält 110,1 g (90 % der Theorie) an ß-Methoximinobuttersäureethylester vom Siedepunkt 76-77°C/ 12 mbar.

c) Beispiel (VII-1)

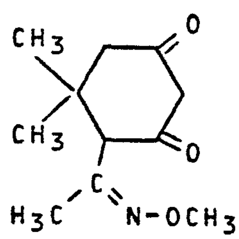

(VII-1)

Man gibt 190 g (1,2 Mol) ß-Methoximinobuttersäureethylester zu einer Lösung von 25,3 g (1,1-Mol) Natrium in 400 ml Ethanol und tropft anschließend 112,8 g (1,15 Mol) Mesityloxid zu. Nach beendeter Zugabe erhitzt man für 5 Stunden auf Rückflußtemperatur, engt dann im Vakuum ein, nimmt den Rückstand in Wasser auf, extrahiert zweimal

mit Dichlormethan, säuert die wäßrige Phase an und extrahiert anschließend mehrmals mit Chloroform. Die vereinigten Chloroform-Phasen werden über Magnesiumsulfat getrocknet, dann im Vakuum eingeengt; der ölige Rückstand kristallisiert durch Verrühren mit Ether. Man erhält 169,1 g (72,8 % der Theorie) an 5,5-Dimethyl-4-(1-methoximinoethyl)-cyclohexan-1,3-dion vom Schmelzpunkt 127-128°C.

In entsprechender Weise und gemäß den allgemeinen Herstellungsangaben erhält man die folgenden Cyclohexan-1,3-dione der Formel (VII):

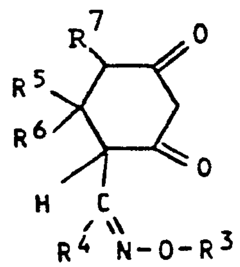

(VII)

Als physikalische Daten sind in der folgenden Tabelle 4 angegeben:

Schmelzpunkt (Fp) in °C
Brechungsindex ($n_D$) /Temperatur in °C/
chemische Verschiebung $\delta$ in ppm bezogen auf $^1$H-NMR
in $CDCl_3$ / ($d^6$-DMSO) *

Tabelle 4:

| Bei-spiel Nr. | $R^3$ | $R^4$ | $R^5$ | $R^6$ | $R^7$ | physikal. Daten |
|---|---|---|---|---|---|---|
| VII-2 | $CH_3$ | $CH_3$ | 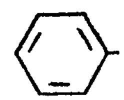 | H | H | Fp. 150 - 153 |
| VII-3 | $CH_3$ | H | $CH_3$ | $CH_3$ | H | 3,86δ($OCH_3$) |
| VII-4 | $CH_3$ | $CH_3$ | 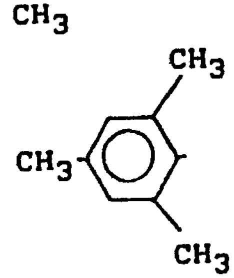 | H | H | Fp. 161-164 |
| VII-5 | $CH_3$ | $CH_3$ | 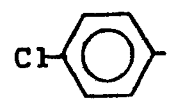 | H | H | 3,50δ($OCH_3$)* |
| VII-6 | $CH_3$ | $n-C_3H_7$ | $CH_3$ | $CH_3$ | H | 3,82δ ($OCH_3$) |
| VII-7 | $CH_3$ | $n-C_3H_7$ | 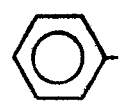 | H | H | 3,71δ($OCH_3$) |

Tabelle 4: (Fortsetzung)

| Beispiel Nr. | $R^3$ | $R^4$ | $R^5$ | $R^6$ | $R^7$ | physikal. Daten |
|---|---|---|---|---|---|---|
| VII-8 | $CH_3$ | $CH_3$ | 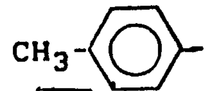 | H | H | 3,69δ($OCH_3$) |
| VII-9 | $CH_3$ | $CH_3$ | 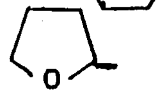 | H | H | 3,59δ($OCH_3$)* |
| VII-10 | $CH_3$ | $CH_3$ | $(CH_3)_3C-$ | H | H | 3,79δ($OCH_3$) |
| VII-11 | $CH_3$ | $CH_3$ | 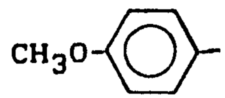 | H | H | Fp. 55 |
| VII-12 | $CH_3$ | $CH_3$ | 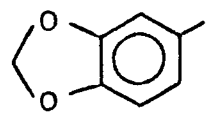 | H | H | Fp. 133 |
| VII-13 | $CH_3$ | $CH_3$ | 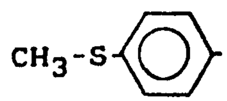 | H | H | |

Tabelle 4: (Fortsetzung)

| Beispiel Nr. | $R^3$ | $R^4$ | $R^5$ | $R^6$ | $R^7$ | physikal. Daten |
|---|---|---|---|---|---|---|
| VII-14 | $CH_3$ | $CH_3$ | 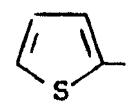 | H | H | 3,58δ $(OCH_3)$* |
| VII-15 | $CH_3$ | $CH_3$ | 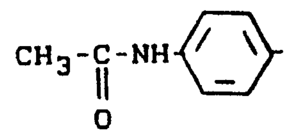 | H | H | Fp. 188-195 |
| VII-16 | $-CH_2-CH=CH_2$ | $CH_3$ | $CH_3$ | $CH_3$ | H | 4,52δ$(OCH_2-)$ |
| VII-17 | $CH_3$ | $CH_3$ | 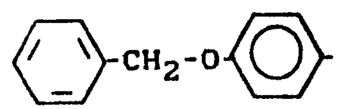 | H | H | Fp. 156 - 158 |
| VII-18 | $-C_2H_5$ | $CH_3$ | $CH_3$ | $CH_3$ | H | 4,04δ $(OCH_2-)$ |
| VII-19 | $CH_3$ | $CH_3OCH_2-$ | $CH_3$ | $CH_3$ | H | 3,32δ $(OCH_3)$ |
| VII-20 | 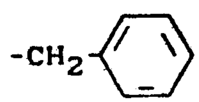 | $CH_3$ | $CH_3$ | $CH_3$ | H | 5,05 δ$(OCH_2-)$ |

Tabelle 4: (Fortsetzung)

| Beispiel Nr. | $R^3$ | $R^4$ | $R^5$ | $R^6$ | $R^7$ | physikal. Daten |
|---|---|---|---|---|---|---|
| VII-21 | $-CH_2-$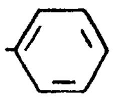 | $CH_3$ | 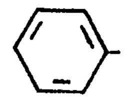 | H | H | 4,91δ($OCH_2$-) |
| VII-22 | $CH_3$ | $CH_3OCH_2$- | 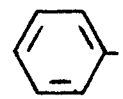 | H | H | 7,19δ (Phenyl) |
| VII-23 | $CH_3$ | $(CH_3)_2CH_2$- | $CH_3$ | $CH_3$ | H | 3,83δ($OCH_3$) |
| VII-24 | $CH_3$ | $C_2H_5$-S-$CH_2$- | $CH_3$ | $CH_3$ | H | 3,82δ($OCH_3$) |
| VII-25 | $CH_3$ | $C_2H_5$ | $CH_3$ | $CH_3$ | H | 3,82δ($OCH_3$) |
| VII-26 | $CH_3$ | $CH_3$ | H | H | H | 3,85δ($OCH_3$) |
| VII-27 | $CH_3$ | n-$C_3H_7$ | H | H | H | 3,79δ($OCH_3$) |
| VII-28 | $CH_3$ | $CH_3$ | $C_2H_5-S-CH(CH_3)-CH_2-$ | H | H | 3,81δ($OCH_3$) |
| VII-29 | $CH_3$ | $CH_3OCH_2$- | H | H | H | 3,33δ($CH_3OCH_2$-) |

Tabelle 4: (Fortsetzung)

| Beispiel Nr. | $R^3$ | $R^4$ | $R^5$ | $R^6$ | $R^7$ | physikal. Daten |
|---|---|---|---|---|---|---|
| VII-30 | $CH_3$ | 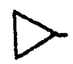 | $CH_3$ | $CH_3$ | H | Fp. 132-133 |
| VII-31 | $CH_3$ | 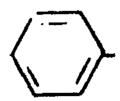$-CH_2-$ | $CH_3$ | $CH_3$ | H | Fp. 146-147 |
| VII-32 | $CH_3$ | $(CH_3)_2CH-CH_2-$ | $CH_3$ | $CH_3$ | H | Fp. 116-118 |
| VII-38 | $-CH_2-$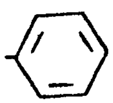 | $CH_3$ | H | H | H | Fp. 120-123 |
| VII-34 | $CH_3$ | $C_2H_5$ | H | H | H | 3,80δ ($OCH_3$) |
| VII-35 | $CH_3$ | $CH_3$ | 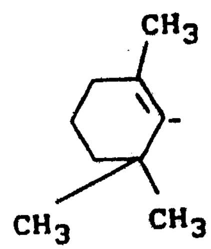 | H | H | Fp. 171-173 |

Tabelle 4: (Fortsetzung)

| Beispiel Nr. | $R^3$ | $R^4$ | $R^5$ | $R^6$ | $R^7$ | physikal. Daten |
|---|---|---|---|---|---|---|
| VII-36 | $CH_3$ | $C_2H_5OCH_2$- | $CH_3$ | $CH_3$ | H | 1,4996 [22] |
| VII-37 | $(CH_3)_2CH$- | $CH_3$ | $CH_3$ | $CH_3$ | H | Fp. 107-111 |
| VII-38 | $CH_3$ | $CH_3$ | 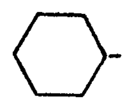 | H | H | 3,82δ($OCH_3$) |
| VII-39 | $CH_3$ | $CF_3$ | $CH_3$ | $CH_3$ | H | 3,93δ ($OCH_3$) |
| VII-40 | $CH_3$ | $CH_3$ | 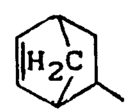 | H | H | 3,81δ ($OCH_3$) |
| VII-41 | $CH_3$ | $CH_3$ | $(CH_3)_2CH$- | H | H | 3,83δ($OCH_3$) |
| VII-42 | $CH_3$ | $CH_3$ | H | $-(CH_2)_6-$ | | Fp. 146-149 |

Anwendungsbeispiele:

In dem folgenden Anwendungsbeispiel wird die nachstehend aufgeführte Verbindung als Vergleichssubstanz eingesetzt:

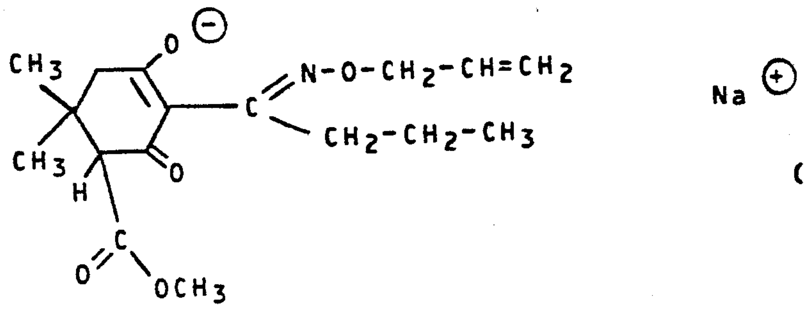

(A)

2-(1-Allyloximino)-butyl-5,5-dimethyl-4-methoxycarbonyl-cyclohexan-1,3-dion-Natriumsalz (bekannt aus DE-OS 24 39 104).

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Wirksamkeit gegenüber dem Stand der Technik zeigt bei diesem Test z.B. die Verbindung gemäß Herstellungsbeispiel 1.

Patentansprüche

1) 2,4-Bis-(alkoximinoalkyl)-cyclohexan-1,3-dione der allgemeinen Formel (I)

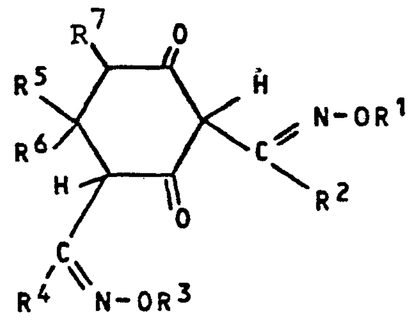

(I)

in welcher

$R^1$ und $R^3$ unabhängig voneinander jeweils für Wasserstoff, Alkyl, Alkenyl, Alkinyl, Alkoxyalkyl, Alkylthioalkyl, Halogenalkyl, Halogenalkenyl, Alkoxycarbonylalkyl, Alkoximinoalkyl, für jeweils gegebenenfalls substituiertes Aralkyl, Aryl oder Heteroarylalkyl oder für Tetrahydropyranyl stehen,

$R^2$ und $R^4$ unabhängig voneinander jeweils für Wasserstoff, Alkyl, Alkenyl, Alkinyl, Alkoxyalkyl, Alkylthioalkyl, Halogenalkyl, Cycloalkyl oder für jeweils gegebenenfalls substituiertes Aryl, Aryloxyalkyl oder Arylthioalkyl stehen,

$R^5$ und $R^6$ unabhängig voneinander jeweils für Wasserstoff, Alkyl, Alkenyl, Alkoxyalkyl, Alkylthioalkyl oder für jeweils gegebenenfalls substituiertes Cycloalkyl, Cycloalkenyl, Aryl, Heteroaryl, Aralkyl, Aryloxyalkyl oder Arylthioalkyl stehen und

0172551

$R^7$ für Wasserstoff steht oder gemeinsam mit $R^6$ für einen zweifach verknüpften Alkandiylrest steht,

sowie deren Metallsalze.

2) 2,4-Bis-(alkoximinoalkyl)-cyclohexan-1,3-dione der Formel (I) gemäß Anspruch 1, in welcher

$R^1$ und $R^3$ unabhängig voneinander jeweils für Wasserstoff, für jeweils geradkettiges oder verzweigtes Alkyl, Alkenyl, Alkinyl, Alkoxyalkyl, Alkylthioalkyl, Halogenalkyl oder Halogenalkenyl mit jeweils bis zu 12 Kohlenstoffatomen in den einzelnen Alkyl- bzw. Alkenyl- oder Alkinylteilen und gegebenenfalls bis zu 9 gleichen oder verschiedenen Halogenatomen stehen, außerdem für jeweils geradkettiges oder verzweigtes Alkoxycarbonylalkyl oder Alkoximinoalkyl mit jeweils bis zu 4 Kohlenstoffatomen in den entsprechenden Alkylteilen oder für jeweils gegebenenfalls einfach oder mehrfach, gleich oder verschieden substituiertes Phenyl oder Phenylalkyl mit gegebenenfalls bis zu 4 Kohlenstoffatomen im geradkettigen oder verzweigten Alkylteil stehen, wobei als Phenylsubstituenten infrage kommen: Halogen, Cyano, Nitro sowie jeweils geradkettiges oder verzweigtes Alkyl, Alkoxy, Alkylthio, Halogenalkyl, Halogenalkoxy, Halogenalkylthio, Dioxyalkylen oder Phenylalkoxy mit jeweils bis zu 4 Kohlenstoffatomen in den einzelnen Alkylteilen und gegebenenfalls bis zu 9 gleichen oder verschiedenen Halogenatomen; ferner für einen gegebenenfalls einfach oder mehrfach, gleich oder verschieden durch Halogen, niederes Alkyl oder niederes Halogenalkyl substituierten 5-gliedrigen oder 6-gliedrigen, über eine Alkylenbrücke mit 1 bis 4 Kohlenstoffatomen verknüpften Heterocyclus mit 1 bis 3 gleichen oder verschiedenen Heteroatomen stehen, wobei als Heteroatome infrage kommen: Stickstoff, Sauerstoff oder Schwefel, und außerdem für Tetrahydropyranyl stehen,

$R^2$ und $R^4$ unabhängig voneinander jeweils für Wasserstoff, für jeweils geradkettiges oder verzweigtes Alkyl, Alkenyl, Alkinyl, Alkoxyalkyl, Alkylthioalkyl oder Halogenalkyl mit jeweils bis zu 12 Kohlenstoffatomen in den einzelnen Alkyl- bzw. Alkenyl- oder Alkinylteilen und mit gegebenenfalls bis zu 9 gleichen oder verschiedenen Halogenatomen, für Cycloalkyl mit 3 bis 7 Kohlenstoffatomen oder für jeweils gegebenenfalls einfach oder mehrfach, gleich oder verschieden substituiertes Phenyl, Phenoxyalkyl oder Phenylthioalkyl mit gegebenenfalls bis zu 4 Kohlenstoffatomen in den geradkettigen oder verzweigten Alkylteilen stehen, wobei als Phenylsubstituenten die bei $R^1$ und $R^3$ genannten infrage kommen,

$R^5$ und $R^6$ unabhängig voneinander jeweils für Wasserstoff, für jeweils geradkettiges oder verzweigtes Alkyl, Alkenyl, Alkoxyalkyl oder Alkylthioalkyl mit bis zu 8 Kohlenstoffatomen in den einzelnen Alkylteilen, für jeweils gegebenenfalls einfach oder mehrfach, gleich oder verschieden durch Halogen oder $C_1$-$C_4$-Alkyl substituiertes Cycloalkyl oder Cycloalkenyl mit jeweils 3 bis 7 Kohlenstoffatomen oder für jeweils gegebenenfalls einfach oder mehrfach, gleich oder verschieden substituiertes Phenyl, Phenylalkyl, Phenoxyalkyl oder Phenylthioalkyl mit gegebenenfalls 1 bis 4 Kohlenstoffatomen in den Alkylteilen stehen, wobei als Substituenten die bei $R^1$ genannten infrage kommen, oder für einen gegebenenfalls einfach oder mehrfach, gleich oder verschieden substituierten 5-gliedrigen oder 6-gliedrigen Heterocyclus mit 1 bis 3 Heteroatomen stehen, wobei als Heteroatome Stickstoff, Sauerstoff oder Schwefel genannt seien und wobei als Substituenten infrage kommen:
Halogen, Cyano, Nitro sowie jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen und

$R^7$ für Wasserstoff steht oder gemeinsam mit $R^6$ für einen geradkettigen oder verzweigten zweifach verknüpften Alkandiylrest mit 3 bis 12 Kohlenstoffatomen steht,

sowie deren Metallsalze.

3) 2,4-Bis-(alkoximinoalkyl)-cyclohexan-1,3-dione der Formel (I) gemäß Anspruch 1, in welcher

$R^1$ und $R^3$ unabhängig voneinander jeweils für Wasserstoff, Methyl, Ethyl, n- oder i-Propyl, n-, i-, s- oder t-Butyl, n- oder i-Pentyl, n- oder i-Hexyl, Undecyl, Allyl, n- oder i-Butenyl, n- oder i-Pentenyl, Propargyl, Methoxymethyl, Ethoxymethyl, Isopropoxymethyl, Methoxyethyl, Ethoxyethyl, Methylthiomethyl, Methylthioethyl, Ethylthioethyl, 2-Chlorethyl, 2-Bromethyl, 2,2,2-Trifluorethyl, 2,2,2-Trichlorethyl, 3-Chlorpropenyl, Methoxycarbonylmethyl, Ethoxycarbonylmethyl, Methoximinomethyl, Methoximinoethyl, Methoximino-2-propyl, jeweils gegebenenfalls ein- bis dreifach, gleich oder verschieden durch Fluor, Chlor, Brom, Cyano, Nitro, Methyl, Methoxy oder Trifluormethyl substituiertes Phenyl oder Benzyl, für einen gegebenenfalls ein- bis dreifach, gleich oder verschieden durch Chlor, Methyl oder Trifluormethyl substituierten, über eine Methylenbrücke verknüpften Heterocyclus der Formel

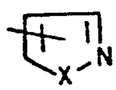 ; 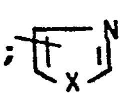 ; 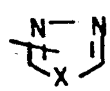 oder 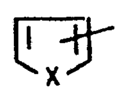

stehen, wobei X jeweils für Sauerstoff oder Schwefel steht, oder für Tetrahydropyranyl stehen,

$R^2$ und $R^4$ unabhängig voneinander jeweils für Wasserstoff, Methyl, Ethyl, n- oder i-Propyl, n-, i-, s- oder t-Butyl, n- oder i-Pentyl, n- oder i-Hexyl, Undecyl, Vinyl, Allyl, n- oder i-Butenyl, n- oder i-Pentenyl, n- oder i-Hexenyl, Propargyl, Methylpropargyl, Dimethylpropargyl, Methoxymethyl, Ethoxymethyl, Isopropoxymethyl, Methoxyethyl, Ethoxyethyl, Methylthiomethyl, Methylthioethyl, Ethylthioethyl, 2-Ethylthio-1-propyl, Trifluormethyl, Dichlorfluormethyl, Difluorchlormethyl, Trichlormethyl, Dichlormethyl, Chlormethyl, 2-Chlorethyl, 2-Bromethyl, 2,2,2-Trichlorethyl, 2,2,2-Trifluorethyl, Cyclopropyl, Cyclobutyl, Cyclopentyl, Cyclohexyl oder jeweils gegebenenfalls ein- bis dreifach, gleich oder verschieden durch Fluor, Chlor, Brom, Cyano, Nitro, Methyl, Methoxy oder Trifluormethyl substituiertes Phenyl, Phenoxymethyl oder Phenylthiomethyl stehen,

$R^5$ und $R^6$ unabhängig voneinander jeweils für Wasserstoff, Methyl, Ethyl, n- oder i-Propyl, n-, i-, s- oder t-Butyl, Vinyl, Allyl, n- oder i-Butenyl, n- oder i-Pentenyl, n- oder i-Hexenyl, Methoxymethyl, Ethoxymethyl, Methoxyethyl, Ethoxyethyl, Methylthiomethyl, Ethylthiomethyl, Ethylthioethyl, Ethylthiopropyl, oder für jeweils gegebenenfalls ein- bis dreifach, gleich oder verschieden durch Fluor, Chlor, Brom, Cyano, Nitro, Methyl, Methoxy, Ethoxy, Dioxymethylen, Benzyloxy, Methylthio, Ethylthio oder Trifluormethyl substituiertes Phenyl, Benzyl, Phenylethyl, Phenoxymethyl, Phenoxyethyl, Phenylthiomethyl, Phenylthioethyl oder Phenylthiopropyl stehen oder für jeweils gegebenenfalls ein- bis dreifach, gleich oder verschieden durch Fluor, Chlor, Brom, Methyl oder Ethyl substituiertes Cyclopropyl, Cyclopentyl, Cyclohexyl, Cyclohexenyl oder Bicyclo-(2,2,1)-heptenyl oder für einen gegebenenfalls ein- bis dreifach, gleich oder verschieden durch Chlor, Methyl oder Trifluormethyl substituierten Heterocyclus der Formel

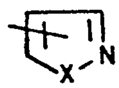 ; 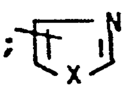 ; 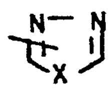 oder 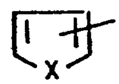

stehen, wobei X jeweils für Sauerstoff oder Schwefel steht und

$R^7$ für Wasserstoff steht, oder gemeinsam mit $R^6$ für 1,3-Propandiyl, 1,4 Butandiyl, 1,5-Pentandiyl oder 1,6-Hexandiyl steht.

4) Verfahren zur Herstellung von 2,4-Bis-(alkoximino-Alkyl)-cyclohexan-1,3-dionen der Formel (I)

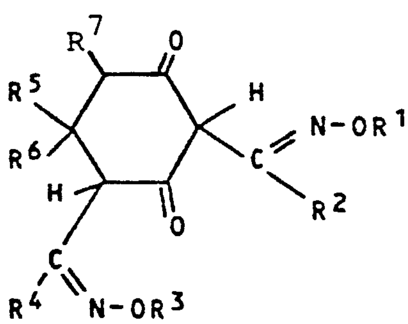

(I)

in welcher

$R^1$ und $R^3$ unabhängig voneinander jeweils für Wasserstoff, Alkyl, Alkenyl, Alkinyl, Alkoxyalkyl, Alkylthioalkyl, Halogenalkyl, Halogenalkenyl, Alkoxycarbonylalkyl, Alkoximinoalkyl, für jeweils gegebenenfalls substituiertes Aralkyl, Aryl oder Heteroarylalkyl oder für Tetrahydropyranyl stehen,

$R^2$ und $R^4$ unabhängig voneinander jeweils für Wasserstoff, Alkyl, Alkenyl, Alkinyl, Alkoxyalkyl, Alkylthioalkyl, Halogenalkyl, Cycloalkyl oder für jeweils gegebenenfalls substituiertes Aryl, Aryloxyalkyl oder Arylthioalkyl stehen,

$R^5$ und $R^6$ unabhängig voneinander jeweils für Wasserstoff, Alkyl, Alkenyl, Alkoxyalkyl, Alkylthioalkyl oder für jeweils gegebenenfalls substituiertes Cycloalkyl, Cycloalkenyl, Aryl, Heteroaryl, Aralkyl, Aryloxyalkyl oder Arylthioalkyl stehen und

$R^7$ für Wasserstoff steht oder gemeinsam mit $R^6$ für einen zweifach verknüpften Alkandiylrest steht,

dadurch gekennzeichnet, daß man

(a) Ketone der Formel (II),

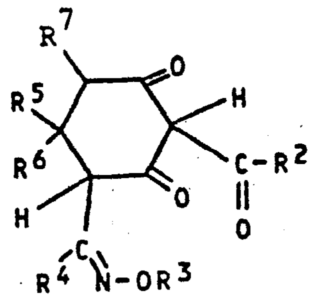

(II)

in welcher

$R^2$, $R^3$, $R^4$, $R^5$, $R^6$ und $R^7$ die oben angegebene Bedeutung haben,

mit Hydroxylamin-Derivaten der Formel (III),

$$H_2N - O - R^1 \qquad (III)$$

in welcher

$R^1$ die oben angegebene Bedeutung hat,

oder mit deren Hydrosalzen, gegebenenfalls in Gegenwart eines Verdünnungsmittels und gegebenenfalls in Gegenwart eines Säurebindemittels umsetzt, oder daß man

(b) die nach Verfahren (a) erhältlichen Oxime der Formel (Ia),

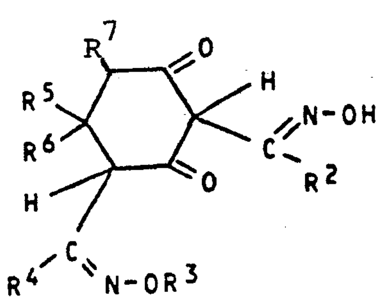

(Ia)

in welcher

$R^2$, $R^3$, $R^4$, $R^5$, $R^6$ und $R^7$ die oben angegebene Bedeutung haben,

mit Alkylierungsmitteln der Formel (IV),

$$R^{1'} - X \qquad (IV)$$

in welcher

$R^{1'}$ für die gleichen Reste wie $R^1$ steht, mit Ausnahme des Wasserstoffrestes und

X für eine elektronenanziehende Abgangsgruppe steht,

gegebenenfalls in Gegenwart eines Verdünnungsmittels sowie gegebenenfalls in Gegenwart eines Säurebindemittels und gegebenenfalls in Gegenwart eines Katalysators umsetzt.

5) Herbizide Mittel, gekennzeichnet durch einen Gehalt von mindestens einem 2,4-Bis-(alkoximinoalkyl)-cyclohexan-1,3-dion der Formel (I) gemäß den Ansprüchen 1 bis 4.

6) Verfahren zur Bekämpfung von Unkraut, dadurch gekennzeichnet, daß man 2,4-Bis-(alkoximinoalkyl)-cyclohexan-1,3-dione der Formel (I) gemäß den Ansprüchen 1 bis 4 auf die Unkräuter oder ihren Lebensraum einwirken läßt.

7) Verwendung von 2,4-Bis-(alkoximinoalkyl)-cyclohexan-1,3-dionen der Formel (I) gemäß den Ansprüchen 1 bis 4 zur Bekämpfung von unerwünschtem Pflanzenwachstum.

8) Verfahren zur Herstellung von herbiziden Mitteln, dadurch gekennzeichnet, daß man 2,4-Bis-(alkoximinoalkyl)-cyclohexan-1,3-dione der Formel (I) gemäß den Ansprüchen 1 bis 4 mit Streckmitteln und/oder oberflächenaktiven Mitteln vermischt.

9) Ketone der Formel (II)

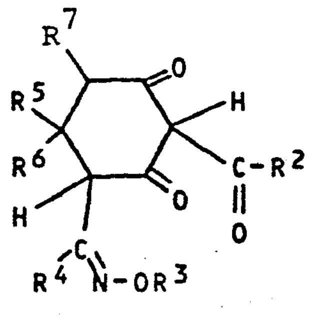

(II)

in welcher

$R^3$ für Wasserstoff, Alkyl, Alkenyl, Alkinyl, Alkoxyalkyl, Alkylthioalkyl, Halogenalkyl, Halogenalkenyl, Alkoxycarbonylalkyl, Alkoximinoalkyl, für jeweils gegebenenfalls substituiertes Aralkyl, Aryl oder Heteroarylalkyl oder für Tetrahydropyranyl steht,

$R^2$ und $R^4$ unabhängig voneinander jeweils für Wasserstoff, Alkyl, Alkenyl, Alkinyl, Alkoxyalkyl, Alkylthioalkyl, Halogenalkyl, Cycloalkyl oder für jeweils gegebenenfalls substituiertes Aryl, Aryloxyalkyl oder Arylthioalkyl stehen,

$R^5$ und $R^6$ unabhängig voneinander jeweils für Wasserstoff, Alkyl, Alkenyl, Alkoxyalkyl, Alkylthioalkyl oder für jeweils gegebenenfalls substituiertes Cycloalkyl, Cycloalkenyl, Aryl, Heteroaryl, Aralkyl, Aryloxyalkyl oder Arylthioalkyl stehen und

$R^7$ für Wasserstoff steht oder gemeinsam mit $R^6$ für einen zweifach verknüpften Alkandiylrest steht.

1o) Cyclohexan-1,3-dione der Formel (VII),

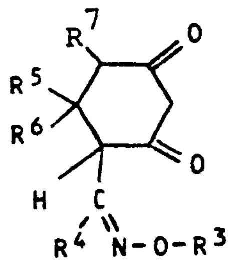

(VII)

in welcher

$R^3$ für Wasserstoff, Alkyl, Alkenyl, Alkinyl, Alkoxyalkyl, Alkylthioalkyl, Halogenalkyl, Halogenalkenyl, Alkoxycarbonylalkyl, Alkoximinoalkyl, für jeweils gegebenenfalls substituiertes Aralkyl, Aryl oder Heteroarylalkyl oder für Tetrahydropyranyl steht,

$R^4$ für Wasserstoff, Alkyl, Alkenyl, Alkinyl, Alkoxyalkyl, Alkylthioalkyl, Halogenalkyl, Cycloalkyl oder für jeweils gegebenenfalls substituiertes Aryl, Aryloxyalkyl oder Arylthioalkyl steht,

$R^5$ und $R^6$ unabhängig voneinander jeweils für Wasserstoff, Alkyl, Alkenyl, Alkoxyalkyl, Alkylthioalkyl oder für jeweils gegebenenfalls substituiertes Cycloalkyl, Cycloalkenyl, Aryl, Heteroaryl, Aralkyl, Aryloxyalkyl oder Arylthioalkyl stehen und

$R^7$ für Wasserstoff steht oder gemeinsam mit $R^6$ für einen zweifach verknüpften Alkandiylrest steht.